(19)

**Europäisches Patentamt**
**European Patent Office**
**Office européen des brevets**

(11) **EP 4 629 143 A1**

(12) **EUROPEAN PATENT APPLICATION**

(43) Date of publication:
08.10.2025 Bulletin 2025/41

(21) Application number: 24305871.6

(22) Date of filing: 03.06.2024

(51) International Patent Classification (IPC):
**G06N 10/60** (2022.01)     **G16C 10/00** (2019.01)
**G16C 20/30** (2019.01)

(52) Cooperative Patent Classification (CPC):
**G06N 10/60; G16C 10/00; G16C 20/30**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **05.04.2024 FR 2403571**

(71) Applicant: **Pasqal S.A.S.**
**91300 Massy (FR)**

(72) Inventors:
• **BARKOUTSOS, Panagiotis**
**91300 Massy (FR)**
• **SOKOLOV, Igor Olegovich**
**91300 Massy (FR)**
• **BOTH, Gert-Jan**
**91300 Massy (FR)**
• **ELFVING, Vincent Emanuel**
**91300 Massy (FR)**

(74) Representative: **De Vries & Metman**
**Overschiestraat 180**
**1062 XK Amsterdam (NL)**

(54) **DETERMINING EXCHANGE-CORRELATION FUNCTIONALS FOR MOLECULAR SYSTEMS USING A QUANTUM COMPUTER**

(57)     A method of determining an exchange-correlation functional for computing electronic properties of a molecular system is described. A hybrid data processor comprising a classical computer and a quantum computer is used to determine the exchange functions. The method may include receiving or determining reference data comprising synthetically computed and/or real-world measured reference electron-orbital features, preferably reference electron densities and associated reference ground state energies, for a plurality of grid points, the reference electron-orbital features being associated with different molecular reference systems; determining a first electron density of a molecular system for which reference electron-orbital features are available; computing a second electron density for each of the grid points based on: the first electron density, an exchange correlction energy computed by a variational quantum model using the first electron density as input; and, a density functional algorithm, preferably a self-consistent density functional algorithm; and, if the difference between the second electron density and the first electron density is smaller than a predetermined delta value, then updating variational parameters of the variational quantum model based on a training loss, wherein a loss function is used to compute the training loss based on the second electron density and a reference electron density respectively.

Fig. 1

**EP 4 629 143 A1**

**Description**

<u>Technical field</u>

The disclosure relates to exchange-correlation functionals for molecular systems, and in particular, though not exclusively, to methods and systems for determining exchange-correlation functionals for molecular systems using a quantum computer and methods and systems for determining electronic properties of a molecular system using such exchange-correlation functionals, and a computer program product for executing such methods.

<u>Background</u>

Density Functional Theory (DFT) has emerged as a major direction of research in the modern quantum chemistry, enabling the efficient prediction of the electronic structure and properties of molecules and materials. It has become the dominant method for solving the Schrodinger equation in physics-based atomistic simulations. DFT's effectiveness largely stems from the ability to express the ground-state density of interacting electrons via a system of non-interacting electrons, the so-called Kohn-Sham system. However, DFT can yield exact ground state energy and electron density only if the *exact* exchange-correlation (XC) functional is provided. Despite the widespread applicability of the technique, the development of accurate and versatile DFT XC functionals remains a challenge. Consequently, DFT faces problems such as underestimation of reaction barriers, bandgaps, and poor handling of degenerate states potentially due to delocalization and static correlation errors.

The universal functional capable of solving the aforementioned problems and capturing the diverse electronic properties of molecules, solids and surfaces remains unknown. In its pursuit, hundreds of approximate XC functionals have been devised. Many of these XC functionals are tailored for certain types of chemical systems and properties thereof, and often require prior knowledge of the targeted data to ensure the success of the method. An additional challenge when constructing such functionals lies in achieving a good balance between accuracy and computational efficiency.

A number of recent studies propose the use of Machine Learning (ML) techniques as a solution for the efficient design of XC functionals that can be used for generic DFT calculations. ML algorithms, ranging from deep neural networks to sophisticated regression models, have demonstrated their potential to unlock hidden patterns in vast datasets of chemical systems, allowing for the estimation of a universal XC functional. This combination of quantum chemistry and artificial intelligence heralds a promising era for XC functional design with many demonstrations for different systems indicating the increase of the reach of the DFT methods. The majority of these ML-based techniques leverage differentiable programming as a widely used paradigm in Deep Learning (DL) that involves optimizing parameters using gradient-based methods.

For example, WO2022/148847 and an associated article by Kirkpatrick et al, Pushing the frontiers of density functionals by solving the fractional electron problem, science 374 (6573) 2021, descibe a scheme to compute exchange-correlation energies of atomic systems using neural networks. Similarly, Li et al., describe in their aticle Kohn-Sham equations as regularizer: building prior knowledge into machine-learned physics (Phys. Rev. Lett. 126, 036401 (2021)), a scheme for computing exchange-correlations energies using neural networkds. Training neural XC functionals based a DFT procedure show improved generalization capabilities in 1D compared to the previous approach however to date useful general XC functionals based on deep neural networks have not been realized yet.

Hence, from the above, it follows that there is a need in the art for improved methods and systems for determining exchange-correlation functionals for computing electronic properties of molecular systems.

<u>Summary</u>

As will be appreciated by one skilled in the art, aspects of the present invention may be embodied as a system, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Functions described in this disclosure may be implemented as an algorithm executed by a microprocessor of a computer. Furthermore, aspects of the present invention may take the form of a computer program product embodied in one or more computer readable medium(s) having computer readable program code embodied, e.g., stored, thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber, cable, RF, etc., or any suitable combination of the foregoing. Computer program code for carrying out operations for aspects of the present invention may be written in any combination of one or more programming languages, including an object-oriented programming language such as Java(TM), Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer, or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described below with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer program instructions. These computer program instructions may be provided to a processor, in particular a microprocessor or central processing unit (CPU), of a general purpose computer, special purpose computer, or other programmable data processing apparatus to produce a machine, such that the instructions, which execute via the processor of the computer, other programmable data processing apparatus, or other devices create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks. Additionally, the Instructions may be executed by any type of processors, including but not limited to one or more digital signal processors (DSPs), general purpose microprocessors, application specific integrated circuits (ASICs), field programmable logic arrays (FP- GAs), or other equivalent integrated or discrete logic circuitry.

The flowchart and block diagrams in the figures illustrate the architecture, functionality, and operation of possible implementations of systems, methods and computer program products according to various embodiments of the present invention. In this regard, each block in the flowchart or block diagrams may represent a module, segment, or portion of code, which comprises one or more executable instructions for implementing the specified logical function(s). It should also be noted that, in some alternative implementations, the functions noted in the blocks may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustrations, and combinations of blocks in the block diagrams and/or flowchart illustrations, can be implemented by special purpose hardware-based systems that perform the specified functions or acts, or combinations of special purpose hardware and computer instructions.

In an embodiment, the embodiments in this disclosure relate to a method of determining an exchange-correlation functional for computing electronic properties of a molecular system using a hybrid data processor comprising a classical computer and a quantum computer, wherein the method may comprise: receiving or determining reference data comprising synthetically computed and/or real-world measured reference electron-orbital features, preferably reference electron densities and associated reference ground state energies, for a plurality of grid points, the reference electron-orbital features being associated with different molecular reference systems; determining a first electron density of a molecular system for which reference electron-orbital features are available; computing a second electron density for each of the grid points based on: the first electron density, an exchange correlction energy computed by a variational quantum model using the first electron density as input; and, a density functional algorithm, preferably a self-consistent density functional algorithm; and, if the difference between the second electron density and the first electron density is smaller than a predetermined delta value, then updating variational parameters of the variational quantum model based on a training loss, wherein a loss function is used to compute the training loss based on the second electron density and a reference electron density respectively.

Hence, the method involves generating a molecular dataset, selecting a quantum model, and optimizing it using specific loss functions. The dataset may be constructed using accurate chemical calculations or experimental results that include the energy and electron density for different molecular systems. A quantum neural network (QNN) comprising a feature embedding block, a parametrized ansatz block, and a measurement block for determing an expectation value of a parametrized observable may then be used. The electron density is loaded into the quantum circuit via the feature map, and a parametrized ansatz may produce a feature vector used to compute the expectation value of an observable to represent the XC energy at a grid point. The DQC-based XC functional may be embedded in a differentiable density functional algorithym, e.g. a differentiable KS-DFT program (e.g., PySCFAD) to estimate the corresponding energy and density. To train the QNN, a cost function may be introduced. In an embodiment, the cost function may be based on the Mean Squared Error (MSE) between the CCSD(T) energies/density and the DQC-based ones.

Gradient-based optimization methods, such as L-BFGS-B or Adam, may be used to vary the parameters and minimize the cost function, however the embodiments in the disclosure are not limited to gradient-based optimizers and may also be applied with any optimizer. The optimized parameters are used to define the quantum-based XC-functional, which can be used for arbitrary molecular structures for enabling more accurate quantum chemistry simulations. The embodiments provide improved XC functionals compared to traditional XC functional design and aims to create reliable quantum-based XC functionals. The quantum-based XC functionals may be used in solving a density functional theory problem in molecular chemistry.

The embodiments use the synergy between QML and DFT for designing XC functionals for computing electronic energies of atomic systems in chemistry. Techniques arising from the QML domain are used to process empirical data from DFT (or general quantum chemistry) calculations within the supervised ML approach to create robust and versatile XC functionals that can be applied in a wider range of problems in the quantum chemistry domain.

In an embodiment, the variational quantum model may be represented by a quantum circuit defining gate operations, the quantum circuit defining a quantum neural network comprising one or more feature maps and one or more parameterized variational circuits.

In an embodiment, the variational quantum model may furhter comprises one or more classical neural networks configured, wherein the updating of the variational parameters includes updating the weights of the classical neural network.

In an embodiment, computing the exchange correlation energy may include: using the one or more feature maps to encode the first electron density at at least part of the grid points in the Hilbert space; executing the quantum circuit on quantum elements of the quantum computer; and, measuring the state of the quantum elements to obtain an exchange correlation energy.

In an embodiment, executing the quantum circuit may include translating quantum operations of the quantum circuit into control signals; controlling the quantum elements of the quantum computer based on the control signals.

In an embodiment, executing the quantum circuit may include: determining classical measurement data by measuring the state of the quantum elements of the quantum computer; and, determining the exchange-correlation energy based on the classical measurement data.

In an embodiment, computing predicted electron-orbital features may include: determining an exchange-correlation potential based on the derivative of the predicted exchange correlation energy with respect to the electron density; and, determining Coulomb and Hartee potentials using the first electron density; computing the second electron-orbital features based on the self-consistent density functional algorithm using the Coulomb and Hartee potentials and the exchange-correlation potential as input.

In an embodiment, the one or more feature maps may be trainable feature maps, the trainable feature maps including one or more unitary operators defining a time evolution over a Hamiltonian, preferably a generator Hamiltonian, applied to the quantum elements of the quantum register, the Hamiltonian evolution being parameterized by variational parameters. Preferably, updating the variational parameters may include updating the variational parameters of the trainable feature maps.

In an embodiment, the quantum circuit may comprise digital quantum gate operations, preferably digital single quantum gate operations, and one or more analog quantum gate operations configured to entangle different quantum elements of the quantum computer and to evolve a Hamiltonian associated with quantum elements in time;

In an embodiment, the method may comprise: if the difference between the second electron-orbital features and the first electron-orbital features is larger than the predetermined delta, computing third electron-orbital features for each of the grid points based on: the second electron-orbital features, a predicted exchange correlction energy computed by the quantum model using the second electron-orbital features as input; and, the density functional algorithm.

In an embodiment, the method may comprise: computing a ground state energy associated with the second electron density; and, further computing the loss value based on the ground state energy and a reference ground state energy associated with the reference electron density.

In an embodiment, at least part of the variational parameters may be adjusted using a classical optimizer, preferably a gradient-based optimizer.

In an embodiment, the one or more electron densities may be encoded based rotation operations and a non-linear encoding function, preferably the non-linear encoding function including a Chebyshev polynomial.

In another embodiment, the data may be encoded into amplitudes of computational basis states of the quantum computer.

In a further embodiment, the quantum model for XC functional may be represented as a projection: $\varepsilon_{XC}(x) = y_\theta(x) = \langle x | y_\theta \rangle$ where the state $|x\rangle$ represents the input data (e.g., electron density) that is encoded in the Hilbert space of the quantum comuter using a Chebyshev or Fourier encoding, and wherein the state $|y_\theta\rangle = U(\theta)|0\rangle$ represents the quantum state after application of a parametrized ansatz $U(\theta)$ on an arbitrary initial state $|0\rangle$.

In an embodiment, the variational quantum model may comprise one or more differentiable quantum circuits (DQCs), the one or more differentiable quantum circuits including one or more derivative features maps for encoding the one or more derivatives of the exchange-correlation functional.

In an embodiment, the quantum computer may be implemented based on at least one of: a neutral atom quantum device, an ion-based quantum device, an optical and photonic quantum device, a superconducting quantum device, a silicon based quantum device, a diamond N-V centre quantum device and/or a gaussian boson sampling device.

In a further aspect, the embodiments may relate to a hybrid data processor for determining an exchange-correlation functional for computing electronic properties of a molecular system, the hybrid data processing comprising a classical computer and a quantum computer, the hybrid data processing being configured to: receiving or determining reference data comprising synthetically computed and/or real-world measured reference electron-orbital features, preferably reference electron densities and associated reference ground state energies, for a plurality of grid points, the reference electron-orbital features being associated with different molecular reference systems; determining a first electron density of a molecular system for which reference electron-orbital features are available; computing a second electron density for each of the grid points based on: the first electron density, an exchange correlction energy computed by a variational quantum model using the first electron density as input; and, a density functional algorithm, preferably a self-consistent density functional algorithm; and, if the difference between the second electron density and the first electron density is smaller than a predetermined delta value, then updating variational parameters of the variational quantum model based on a training loss, wherein a loss function is used to compute the training loss based on the second electron density and a reference electron density respectively.

The embodiments may also relate to a computer program or suite of computer programs comprising at least one software code portion or a computer program product storing at least one software code portion, the software code portion, when run on a hybrid data processing system comprising a classical computer and a quantum computer comprising a hardware quantum computer, being configured for executing the method steps according any of the above-described embodiments.

Brief description of the drawings

**Fig. 1** depicts a hybrid computer processor comprising a classical computer and a quantum computer;

**Fig. 2A** and **2B** illustrates different quantum models for implementing quantum XC functionals;

**Fig. 3** illustrates illustrates classical MPL-based neural network architectures for implementing classical XC functionals;

**FIG. 4A** and **4B** depicts a process for computing electronic properties of a moleculair system using a hybrid computing system according to an embodiment;

**Fig. 5** depicts a flow chart for determining XC functionals for moleculair systems according to an embodiment;

**Fig. 6** depicts the process of computing an electron density based on a self-consistent density functional algorithm according to an embodiment;

**Fig. 7A** and **7B** depict a scheme for executing differentialbe quantum circuits (DQCs) using a quantum computer;

**Fig. 8A-8C** depict quantum circuits which may be used in the embodiments in this disclosure;

**Fig. 9A** illustrates depict another scheme for executing differentiate quantum circuits (DQCs) using a quantum computer;

**Fig. 10** depicts experimental regarding computation of electronic states of a molecular system using an density functional algorithm based on classical XC functions;

**Fig. 11A-11C** depict experimental regarding computation of electronic states of a molecular system using an density functional algorithm based on quantum-enhanced XC functions;

**Fig. 12** schematically depicts a hardware-level schematic illustrating the application of logical operations to qubits using a quantum circuit;

**Fig. 13** schematically depict hardware-level schematics illustrating the application of logical operations to qubits using a quantum circuit;

**Fig. 14** schematically depicts a quantum circuit based on a digital-analog implementation;

**Fig. 15** schematically depicts a quantum circuit based on a further digital-analog implementation;

**Fig. 16** schematically depicts a quantum circuit based on another digital-analog implementation;

**Fig. 17** schematically depicts a quantum circuit based on yet another digital-analog implementation;

**Fig. 18** schematically depicts a quantum circuit based on a further digital-analog implementation.

Description of the embodiments

The embodiments in this disclosure generally relate to improved schemes for efficient training of quantum models as XC functionals for computing electronic properties of molecular systems. Classical neural network schemes for determining XC functions that are used in density functional algorithms for computing electronic propertis for molecular system so far have not provided good results. The embodiments in this disclosure use quantum models to determine exchange-correlation functionals that are used in density functional schemes for quantum chemistry. The embodients further describe methods and systems for determining exchange-correlation functions for molecular systems using a quantum computer and methods and systems for determining electronic properties of a molecular system using quantum models representing exchange-correlation functions.

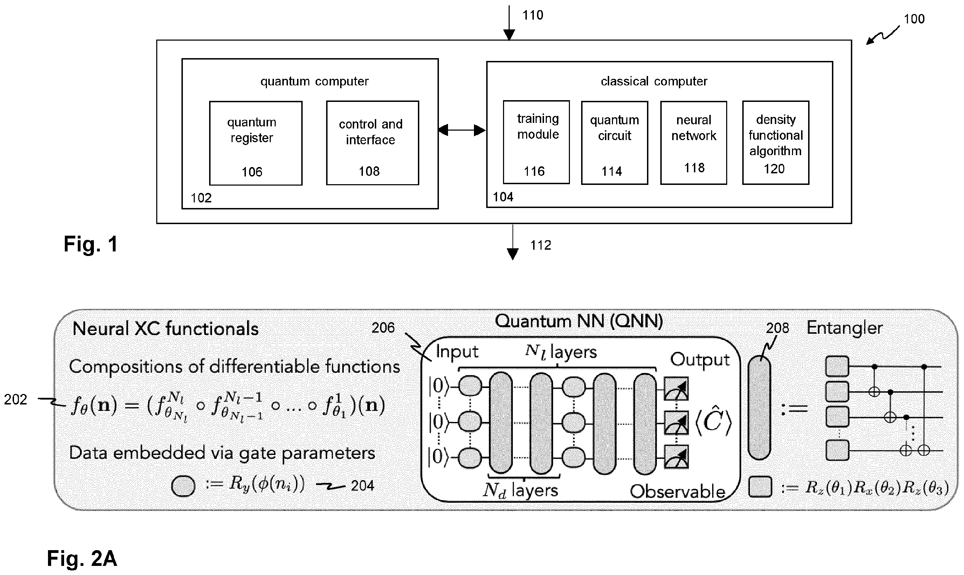

The quantum models described with reference to the embodiments in this application may be executed by a hybrid computer system comprising a quantum computer and a classical computer. **Fig. 1** depicts an example of such hybrid computer system comprising a classical computer **102** comprising one or more classical processors and a quantum computer system **104** wherein the classical computer may be configured to train a quantum model that is executed on the quantum computer as a functional that is used in a so-called density theory algorithms which are used to compute electron-orbital features, such as an electron density and a ground state energy of a molecular system. As shown in the figure, the quantum computer system may comprise one or more quantum registers **106,** e.g. a gate-based qudit quantum register. Such quantum register may also be referred to as a quantum processor or a quantum processing unit (QPU). The quantum register may be implemented based on one of the well-known quantum hardware platforms including but not limited to superconducting circutis, quantum dots, neutral atoms, ions, optical qubits or NV centers.

The system may further include a memory storage for storing a representation of a quantum circuit **114.** A quantum circuit may represent a set of instructions to be executed on the quantum computer. The instructions are transformed into control signals for executing gate operations or unitaries on the qubits of the quantum computer, wherein the control signals are sent by a classical computer to the quantum computer. In practice, the execution of a quantum circuit may involve a sequence of operations executed on the quantum registers by a controller system **108** comprising input output (I/O) devices which form an interface between the quantum register and the classical computer. For example, the controller system may include an optical and/or electromagnetic pulse generating system for generating pulses, e.g. optical, voltage and/or microwave pulses, representing gate operations to be applied to the qudits of the quantum register in accordance with the quantum circuit.

The controller system may include readout circuitry for readout of the qudits. At least a part such readout circuitry may be located or integrated with the chip that includes the qudits. The system may further comprise a (purely classical information) input **110** an (purely classical information) output **112.** The input and output may be part of an interface, e.g. an interface such as a user interface or an interface to another system. Input data may include training data associated with molecular systems. The training dataset may comprise reference data, in particular reference electron-orbital features such as reference electronic densities and associated energies, e.g. ground state energies, of atomic and/or molecular systems. The reference data may include synthetic data reference data which may be computed using accurate ab initio methods such as full configuations interation (FCI) calculations. Further, the reference data may include experimental data, regarding electron-orbital features such as electron densities and ground state energies for the different atomic and/or molecular systems. Other information may include information about the quantum model, including parameters and/or conditions that are needed for training process.

The input data may be used by the system to classically calculate values, e.g. parameter settings, which may be used to initialize a quantum circuit that is implemented on the quantum processor. Similarly, output data may include loss function values, sampling results, expectation values, optimization convergence results, optimized quantum circuit parameters and hyperparameters, and other classical data. The system may include a training module **116** that is configured to train a quantum model based on gate operations of a parameterized quantum circuit **114** which may be stored in a memory of the system. In some embodiment, the trainable quantum model may represent a quantum neural network (QNN). The system may also include one or more classical neural networks which may be used in combination with the QNN to form a hybrid quantum neural network. Information about the quantum circuit and training data may be provided via input **110** to the classical computer.

The embodiments in this disclosure describe density functional schemes, such as the Kohn-Sham Density Functional Theory (KS-DFT) scheme, that use quantum models that are trained as XC functionals. More generally, the embodiments in this disclosure describe quantum machine learning (QML) schemes for training XC functionals and ways of using (embedding) such neural XC functionals in a density functional scheme, e.g. the KS-DFT scheme, for computing electronic properties of molecular systems.

Hence, the system in **Fig. 1** may include a density functional algorithm **120** that uses a trained quantum model or a trained hybrid quantum model to compute electronic properties such as electron-orbital features of a new molecular system.

Electron-orbital features such as the electronic ground state of an atomic or molecular system may be determined based on self-consistent resolution of the KS equations,

$$\hat{H}[n](\mathbf{r})\phi_i(\mathbf{r}) = \epsilon_i\phi_i(\mathbf{r}), \qquad (1)$$

The Hamiltonian operator $\hat{H}$ may be defined as $\hat{H}[n](r)\phi_i(r) = -\nabla^2/2 + v_{KS}[n](r)$ wherein $n(r)$, is the electron density which may be define as the sum of probability densities over all $N_{occ}$ occupied electron-orbitals,

$n(r) = \sum_{i=1}^{N_{occ}}|\phi_i(r)|^2$ ,. Here, $\phi_i$ may represent an electron-orbital, in particular a one-electron KS orbital, with corresponding energy $\varepsilon_i$. The KS potential $v_{XC}$ may be defined based on the external ionic Coulomb potentials $v(r)$, the Hartree potential $v_H[n](r)$ and the XC potential: $v_{XC}[n](r)v_{KS}[n](r) = v(r) + v_H[n](r) + v_{XC}[n](r)$. The XC potential may be computed as the functional derivative of the XC energy, $E_{XC}$, with respect to the electron density, i.e. $v_{XC}[n](r) = \delta E_{XC}[n]/\delta n(r)$, wherein the XC energy $E_{XC}$ is defined as

$$E_{XC}[n] = \int d\mathbf{r}\epsilon_{XC}[n](\mathbf{r})n(\mathbf{r}), \qquad (2)$$

and wherein $\varepsilon_{XC}[n](r)$ is the XC energy per electron. Hence, in this scheme a XC function may be used to compute energies associated with electron-orbitals wherein the integral over all energies may defined the XC energy. The total energy of the atomic or molecular system may then be obtained as the sum, of the kinetic energy $T_S[n]$, the external potential energy $V[n]$, the Hartree energy $E_H[n]$ and the XC energy $E_{XC}[n]$: $E[n] = T_S[n] + V[n] + E_H[n] + E_{XC}[n]$.

Given a finite basis set $\{\xi_i\}_{i=1}^{N_b}$, a linear combination of $N_b$ basis functions, $\phi_i(r) = \sum_{j=1}^{N_b} p_i^j \xi_i(r)$, may be optimized to produce suitable electron-orbitals $\phi$. This involves the solution of Roothan's equation defined as

$$\mathcal{F}[n]\mathbf{p}_i = \epsilon_i S\mathbf{p}_i, \qquad (3)$$

where $p_i = (p^1,...,p^{N_b})^T$ are the electron-orbital coefficients, $S_{ij} = \int d\mathbf{r}\xi_i(\mathbf{r})\xi_j^*(\mathbf{r})$ is an overlap matrix and $\mathcal{F}[n]$ is the Fock matrix as a functional of the electron density profile $n$, wherein elements of the Fock matrix may be defined as

$\mathcal{F}_{ij} = \int d\mathbf{r}\xi_i(\mathbf{r})\hat{H}[n]\xi_j^*(\mathbf{r})$ .

Eq. (3) may then be solved, e.g., numerically, by an eigen decomposition of the Fock matrix to obtain the orbital coefficients $\{\mathbf{p}_i\}$ that correspond to an electron density profile $n$. Hence, given a basis of electron-orbitals, a corresponding electron density may be determined based on a self-consistent iterative calculation. Self-consistency may be achieved when the electron density no longer changes within a specified tolerance for a number $N_{KS}$ of KS iterations (solutions of Eq.(3)). The energy E associated with the computed electron density can then be compared to validated data, e.g. experimental and/or computed reference data. Here, the quality of the computed electron density and energy may dependent on the choice of the functional used to represent $E_{XC}[n]$. If the exact XC functional was known, the Hohenberg-Kohn theorem guarantees that the self-consistent solution yields the exact ground-state energy and density.

In an embodiment, a quantum model, e.g. a trainable quantum neural network QNN, may be trained according to Eq. (2), so that it represents a XC functional. The quantum model may represent a differentiable function $f_\theta: (n, r) \to \epsilon_{XR}(\mathbf{r})$ with trainable parameters $\theta$. Hence, in this embodiment, the trained quantum model $f_\theta(n,\mathbf{r})$ representing an XC functional may be used to determine an energy for a given machine learned XC energy.

**Fig. 2A** illustrates a quantum model representing a XC functional as a combination of differentiable functions. As shown in the figure a quantum model representing an XC functional may be implemented as one or more QNNs and - in some embodiments - one or more classical NNs to form a composition of differentiable functions **202,** which can be implemented based on a quantum neural network (QNN) **204.** The QQN may be represented as a quantum circuit including "layers" of gate operations including single gate operations **206** associated with one or more feature maps which may be used for encoding input data in the Hilbert space of the quantum computer. The encoding of the input data may be realized via the the gate parameters of the gate operations of the feature map. The quantum circuit may further include one or more variational quantum circuits **208** for enabling the qubits to interact with each other.

The XC energy may be expressed in general as $E[n] \approx E_{XC}^{\theta}[n] = \int dr f_\theta(n, \mathbf{r}) n(\mathbf{r})$, where $E_{XC}^{\theta}[n]$ approximates the exact correlation energy $E_{XC}[n]$. A so-called integration grid may be used to compute the integral of equation (2) . The integration grid may be a finite numerical integration grid of $N_{grid}$ grid points. The grid points may be grid points of any grid scheme suitable for three-dimensional numerical integration. In some embodiments, the plurality of grid points may include grid points on a real-space quadrature grid. Embodiments of specific grid schemes include the Treutler, Mura-Knowles, Delley, and Gauss-Chebyshev schemes.

The grid points may be associated with a coordinate system, e.g. a Cartesian coordinate system or the like. In an embodiment, a grid generation algorithm based on the atom-centered grids (ACGs) approach may be used. This algorithm is configured to generate a set of radial and angular grids for each atom in a molecule. In an embodiment, a radial grid may be designed to be denser near the nucleus and sparser in the outer regions, while the angular grids may be designed to be denser near the poles and sparser near the equator.

In an embodiment, the XC energy may be computed as an integral over a neural functional $f_\theta$ at each of the grid points:

$$E_{XC}[n] \approx E_{XC}^{\theta,1}[n] = \sum_{i=1}^{N_{grid}} w_i f_\theta(n, \mathbf{r}_i) n(\mathbf{r}_i), \qquad (4)$$

Here, $w_i$ defined grid weights $\{w_i\}_{i=1}^{N_{grid}}$ which may be computed for each grid type and molecular system. The article by Dsgupta et al, Standard grids for high-precision integration of modern density functionals: SG-2 and SG-3, Journal of Computational Chemistry 38(12), 2017 describes gridpoints that may be used for the embodiments in this closure. Here, $\{\mathbf{r}_i\}_{i=1}^{N_{grid}}$ denote Cartesian coordinates associated with the grid points so that $n(\mathbf{r}_i)$ defines an electron density at a grid point coordinate $\mathbf{r}_i$. The computation of the correlation exchange energy according to equation (4) may be referred to as *local energy embedding* since it involves computation of a local XC energy density $\varepsilon_{XR}(\mathbf{r})$ at each grid point.

In another embodiment, the total XC energy may be determined directly using a neural XC functional $F_\theta[n]$, bypassing the integration as explained above with refeence to equation (4), as

$$E_{XC}[n] \approx E_{XC}^{\theta,2}[n] = F_\theta[n], \qquad (5)$$

where during training the neural XC functional $F_\theta[n]$ learns implicitly the relation between the XC energy and the grid points during training through $\{n_i\}_{i=1}^{N_{grid}}$ input density values provided to the input of the neural XC functional. Hence, in this embodiment, a neural network is trained to receive $N_{grid}$ input density value and to compute the XC energy. This approach may be referred to as the *global energy embedding* since the model requires the whole density profile to infer the total XC energy. The local and the global energy embedding (represented by Eqs. (4) and (5)), provide distinct ways of using a trained quantum model as an XC functional in a density functional scheme for computing electronic properties of a molecular system. Despite avoiding grid integration in the global energy embedding, both approaches (local and global) require the computation of the local XC potential $v_{xc}[n](\mathbf{r})$ at every grid point because the local XC potential is used to construct the Fock operator as defined by Eq. 3, which shows that the local XC potential is in the Hamiltonian and Fock operator is the integral of that Hamiltonian with the atomic orbitals.

The quantum model representing an XC functional may be implemented as one or more QNNs and - in some embodiments - one or more classical NNs to form a composition of differentiable functions,

$$f_\theta(n, \mathbf{r}) = (f_{\theta_{N_l}}^{N_l} \circ f_{\theta_{N_l-1}}^{N_l-1} \circ \ldots \circ f_{\theta_1}^1)(n, \mathbf{r}), \qquad (6)$$

comprising trainable parameters $\theta = (\theta_{N_l}, \ldots, \theta_1)$ and $N_l$ the total number of layers of the model. When combining a QNN with one or more classical NNs the model may be referred to as a hybrid quantum model. As described above, either trained quantum models for local embedding or quantum models for global energy embeddings may be used in a density fuctional scheme, such as KS-DFT, for computing electronic properties of a molecular system.

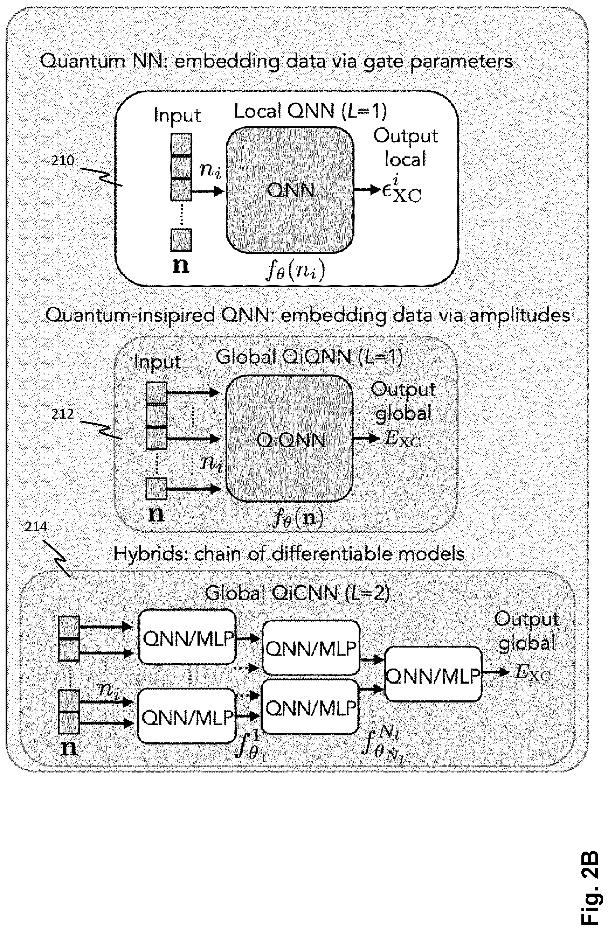

**Fig. 2B** illustrates different quantum neural network (QNN) architectures for implementing quantum XC functionals. The quantum models may be characterized by various degrees of locality that represent the number of electron density points provided at input of the quantum model. The architectures include a local QNN **210** wherein data are embedded via the gate parameters of the feature map into the QNN, a global QiQNN **212** wherein data are embedded in the QNN using an amplitude encoding scheme and a hybrid quantum model **214,** in which a QNN is combined with a classical NN, e.g. an MPL-based NN, to form a QNN/MPL model. QNN/MPL models may be combined to form a network of QNN/MLP models.

The local QNN **210** as depicted in **Fig. 2B** may be used to represent fuctionals $f_\theta^{local} : n_i \longrightarrow \epsilon_{XC,i}^\theta$, where the quantum model only has access to a single local density information value at a grid point. To compute the total XC energy, the XC functional may be applied in succession to each point of the $N_{grid}$ points. Such function may be embedded in the density functional scheme using Eq. (4). In another embodiment, a *L-local* quantum model may be used, which may be represented by the functions

$$f_\theta^{local} : \{n\}^L \to \epsilon_{XC,i}^\theta, \qquad (7)$$

where the quantum model has access to only $L$ neighbouring density points (kernel size) at a time. Hence, to compute the XC energy, the quantum model is applied in succession on, e.g., $\lceil N_{grid}/L \rceil$ points. Similarly to the one-local quantum models this quantum model may be used in the DFT scheme based on Eq. (4).

In further embodiment, global models **212** and **214** may be used, which are configured to receive the whole electron density, i.e. all electron density values at all grid points,

$$F_\theta^{global} : \{n\}_{i=1}^{N_{grid}} \to E_{XC}^\theta, \qquad (8)$$

so that the quantum model can be trained to represent the whole integrated XC energy. A global quantum model may be used, which can be embedded in in the DFT scheme using Eq. (5).

In other embodiment, the global quantum model may (also) be embedded in the DFT scheme using Eq. (4) as

$$f_\theta^{global} : \{n\}_{i=1}^{N_{grids}} \to \{\epsilon_{XC,i}^\theta\}_{i=1}^{N_{grids}}, \qquad (9)$$

wherein the local XC energy depends on the whole electron density.

Known classical models for computing exchange-correlation energies of atomic systems, as e.g. described in WO2022/148847, may be based on a Multilayer Perceptron (MLP) model, wherein the layers are given by $f_{\theta_i}^k(n, \mathbf{r}) = \alpha(A\mathbf{n} + \mathbf{b})$ including activation function $\alpha$ and trainable matrix $A$ and vector $\mathbf{b}$. Given enough layers, a MLP model would be theoretically capable of representing any function according to the universal approximation theorem. However, practical use of an MLP model is hindered by the large number of parameters and the lack of inductive biases. This leads to overfitting on the training data and poor generalization to data outside the training set. MLPs are used in known schemes for computing exchange-correlation energies of atomic systems using classical neural networks. In this disclosure, such classical neural networks are used for comparative studies involving quantum models and hybrid quantum models as described with reference to the embodiments in this disclosure.

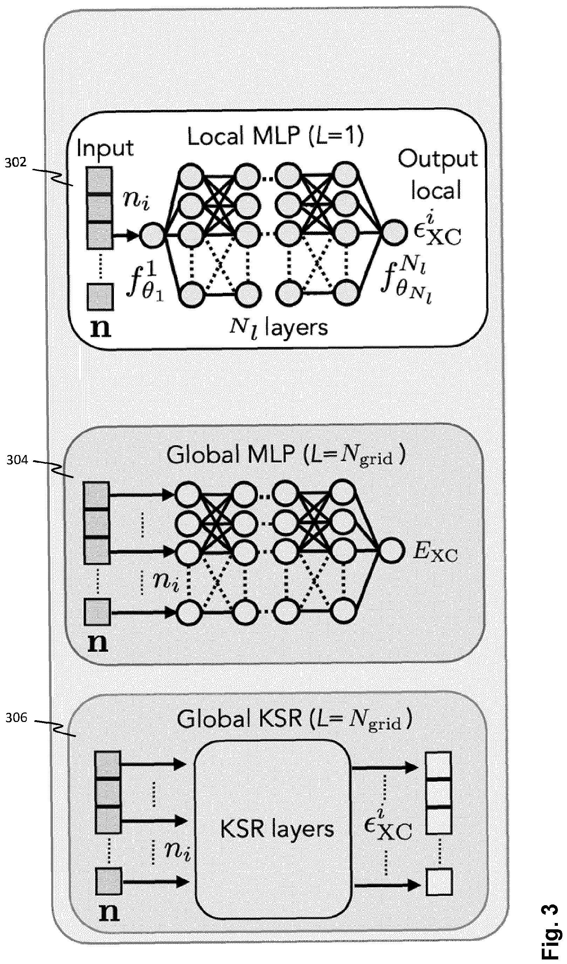

Classical MLP models that are used for these comparative studies are depicted in **Fig. 3.** As shown in the figure, depending on their implementation the models may have different number of inputs and $N_l$ layers consisting of $N_n$ neurons, wherein the Local MLP (LMLP) model may be defined as

$$f_\theta^{\mathrm{LMLP}} : n_i \to \epsilon_{\mathrm{XC},i}^\theta, \qquad (10)$$

using Eq. (7), where the model function has access only to the local density information and is applied on $N_{grid}$ points. **Fig. 3** shows an example of a local MPL model **302** (L=1). This function may be embedded in DFT via Eq. (4). In a similar way, $L$-local MLP, where not a single density value $n_i$ but a collection of $L$ neighbouring densities $\{n_i\}^L$ are taken as input, may be defined. MPL model **304** of **Fig. 3** shows an global MLP (GMLP) neural network which may be defined as a functional of the whole density,

$$F_\theta^{\mathrm{GMLP}} : \{n\}_{i=1}^{N_{\mathrm{grid}}} \to E_{\mathrm{XC}}^\theta, \qquad (11)$$

using Eq. (8). Ths model may be embedded in the DFT theory using Eq. (5).

More elaborated classical NNs may include the Global Kohn-Sham Regularizer (GKSR) model as described in the article by Li et al, Kohn-Sham equations as regularizer: building prior knowledge into machine-learned physics (Phys. Rev. Lett. 126, 036401 (2021). The GKSR model is currently one of the best models for computing the XC energy. The GKSR model combines local and global convolutional layers, and incorporates inductive biases such as a self-interaction gate and a negativity transform, addressing common issues like self-interaction errors and imposing the negativity condition on the XC energy. In an implementation, the GKSR model may be represented by the function:

$$f_\theta^{\mathrm{GKSR}} : \{n\}_{i=1}^{N_{\mathrm{grids}}} \to \{\epsilon_{\mathrm{XC},i}^\theta\}_{i=1}^{N_{\mathrm{grids}}} \qquad (12)$$

using Eq. (9). Despite representing a global functional, it is embedded in DFT via Eq. (4) unlike GMLP as defined above. This allows comparision of the results of embedding these XC models in DFT using both Eq.(4) (local/global) and Eq.(5) (global). As already described above, useful general XC functionals based on trained classical neural network have not been obtained yet. In other words, useful general XC functions for computing electronic properties of molecular systems cannot be achieved based on current classical neural network models.

The embodiments in this application aim to address this problem by using a quantum model, such as a quantum neural network (QNN) model, to learn XC functionals, wherein a quantum model may be represented as a quantum circuit (which may include further (sub) blocks of quantum circuits) comprising qubit operations which need to be executed on qubits or qudits of a quantum coputer. As will be described below in more detail, different quantum models may be used for implementing the embodiments described in this application this application.

Hence, in an embodiment, XC functionals may be learned by optimizing a quantum model, e.g. training a quantum neural network (QNN), comprising blocks of quantum circuits. These blocks may include a feature map block $U_f$ for embeddeding real-world data (i.e. electron-orbital features such as electron densities) into the Hilbert space of the quantum register, a parameterized quantum circuit block (sometimes referred to as an ansatz $U_a$) which may be configured to act on the embedded data using single and multiple qubit operations. Further, a measurement block may be used to determine the expectation value of an observable representing an XC energy. Typically, the QNN may be implemented on a $N_q$-qubit quantum register that is initialized at some initial state 10). For example, in an embodiment, the zero computational basis state for the qubits may be used as the initial state.

A feature map may be used to embed the data (e.g., electron-orbital features such as the electron density $n$) in the Hilbert space, $U_f(n)|0\rangle$ in a differentiable manner so that the XC potential $v_{XC}$ may be computed. As will be described below in more detail, different feature map architectures may be used. In an embodiment, the feature map may be used to embed one or more discretized electron-orbital features, such as one or more discretized electron density values $\{n_i\}_{i=1}^{N_{grid}}$ in the QNN as gate parameters of the feature map. In an embodiment, the feature map may be a trainable feature map.

The parameterized block $U_a(\theta_a)$ having trainable parameters $\theta_a$ may be used to act on the embedded data with local qubit operations and entangling gates. Many different feature maps and/or variational quantum circuit blocks blocks may be used to train a quantum model to efficiently represent an XC functional. Embodiments of such feature maps and variational DQC blocs are disclosed in this application.

In an embodiment, the output of the model may be extracted based on projective measurements (typically referred to as "shots") of an cost operator. In an embodiment, a cost function may be based on Pauli operators. For example, the cost function may be expressed as $\hat{C} = \sum_{i=1}^{N_o} \theta_c^i \hat{O}_i$, where $\theta_c$ are fixed or trainable parameters associated to Pauli operators $\hat{O} \in \{I, \hat{X}, \hat{Y}, \hat{Z}\}^{\otimes N_q}$. In an embodiment, the cost operator may be configured as the total magnetization

$$\hat{C} = \sum_{i=1}^{N_q} \hat{Z}_i$$ , which was shown to avoid cost-function-induced barren plateaus.

In this embodiment, a complete representation of the quantum model may be given by the following expression

$$f_\theta^q(n) = \langle 0|U^\dagger(n,\theta)\hat{C}U(n,\theta)|0\rangle, \qquad (13)$$

where $$U(n,\theta) = \prod_{l=1}^{N_l}\left(\prod_{d=1}^{N_d} U_a^{l,d}\left(\theta_a^{l,d}\right)U_f^l(n)\right)$$ is the general representation of the model with $N_d$ ansatz layers and $N_l$ total repetition of ansatz and feature maps, which may implement the data re-uploading strategy. Repetitions of layers may be used to augment the expressivity of the model in order to provide a good approximate of a general XC function.

In an embodiment, the so-called Hardware-Efficient Ansatz (HEA) known from variational quantum eigensolver (VQE) algorithms that are used in quantum chemistry applications may be used as an ansatz $U_a$ layer. The structure of the HEA may be used to represent a general quantum model. The HEA may comprise one or more blocks of single-qubit rotations (i.e., $$R_z(\theta) = e^{-\frac{i}{2}\theta\hat{Z}}$$ ). The single-qubit rotations may be placed on qubits of a quantum register with distinct angles $\theta_a$,

$$U_{\text{sqr}}(\theta_a) = \prod_{i=1}^{N_q} e^{-\frac{i}{2}\theta_{a,x}^i \hat{Z}} e^{-\frac{i}{2}\theta_{a,y}^i \hat{X}} e^{-\frac{i}{2}\theta_{a,z}^i \hat{Z}}, \qquad (C1)$$

which enable the representation of most general rotation on a Bloch sphere. The HEA may further comprise one or more entanglement blocks. The entanblement blocks may include an arbitrary pattern of (parametrized) multi-qubit gates that introduce interactions between qubits. For example, in an embodiment, an entanglement block include a plurality of CNOT operations

$$U_{\text{ent}} = \prod_{(i,j)\in S} \text{CNOT}(i,j) \qquad (C2)$$

wherein S represents an ordered set of pairs of qubit indices. In an embodiment, a CNOT operation on qubit pair $i, j$ in equation C2 may be defined as $$CNOT(i,j) = e^{-\frac{i}{4}(I_i - \hat{Z}_i)(I_j - \hat{X}_j)}$$ . In an embodiment, an "alternate ladder" set of qubit pairs may be used. Such alternate ladder may be defined as: $S_{al} = S_1 \cup S_2$ with $S_1 = ((0,1),(2,3),...,(N_q - 1, N_q))$ and $S_2 = ((1, 2), (3, 4), ... , (N_q - 2, N_q - 1))$.

In an embodiment, a parametrized entanglement layer may be defined based on a sequence of parameterized ZZ gates. For example, a parameterized entanglement layer may be defined as $$U_{ent}^{RZZ}(\theta_{ent}) = \prod_{(i,j)\in S} R_{ZZ}^{i,j}\left(\theta_{ent}^{i,j}\right) = \prod_{(i,j)\in S} CNOT(i,j)e^{-\frac{i}{2}\theta_{ent}^{i,j}\hat{Z}} CNOT(i,j)$$ , where $R_{ZZ}^{i,j}$ represents the parametrized ZZ gate between qubits. This allows the model to cancel interaction ( $$\theta_{ent}^{i,j} = 0$$ ) between qubits if necessary.

A feature map may be designed to provide expressiveness to a trained quantum model representing an XC functional. In an embodiment, electron-orbital features such as electron energy densities may be encoded in the parameters (e.g. angles) of a quantum circuit, typically a feature map. In another embodiment, the data may be encoded into amplitudes of computational basis states $$\{|i\rangle\}_{i=1}^{2^{N_q}}$$ . A general angle embedding unitary may be represented by a set of feature maps that may be expressed as

$$U_f(\boldsymbol{n}) = \bigotimes_{k=1}^{N_{\text{grid}}} e^{-\frac{i}{2}\varphi(n_k)\hat{G}_k}, \qquad (C4)$$

with generators $\hat{G}$ and a data transformation function $\varphi: \mathbb{R} \rightarrow \mathbb{R}$. In an embodiment, at least part of the generators may be defined as multi-qubit operators. Non-limiting examples of feature maps that may be used to embed data in quantum circuits include product feature maps, Chebyshev feature maps and amplitude data encoding schemes.

In an embodiment, a 1-local product feature map may be used. The fully local version (1-local) of the product feature map may be configured to embed the data value $n_k$ in the rotation angles of $R_y$ gates ($\hat{G}_l = \hat{Y}_l$) of all qubits as

$$U_{f,\mathrm{pr}}^{(1)}(n_k) = \bigotimes_{l=1}^{N_q} e^{-\frac{i}{2} n_k \hat{Y}_l}. \qquad (C5)$$

In another embodiment, a 1-local Chebyshev tower feature map may be used, which utilizes an efficient polynomial basis and which is defined by

$$U_{f,\mathrm{ch}}^{(1)}(\boldsymbol{n}) = \bigotimes_{k=1}^{N_q} e^{-k \cdot \arccos(n_k) \hat{Y}_k}. \qquad (C6)$$

In yet a further embodiment, a plurality of data points (input data) may be encoded by a feature map. For example, in an embodiment, an $L$-local version of a product feature $U_{f,pr}$ may be realized by embedding different data points into single-qubit gates angles associated with available qubits. Here, the parameter $L$ represents the number of data points that can be encoded in the circuit simultaneously. Since the feature maps presented in this disclosure can embed at most $1 \le L \le N_q$ data points, there are $N_{grid}/L$ different embeddings for grid points to perform (model executions). These emboddigns can be executed in parallel since they are independent.

In a further embodiment, amplitude encoding may be used for mapping the density vector $\boldsymbol{n}$ to a quantum state as

$$|\boldsymbol{n}\rangle = U_{f,a}^{(N_{grid})}(\boldsymbol{n})|0\rangle$$
.

A final quantum state $|\psi\rangle$ may be prepared by repetitive applications of one or more feature map blocks and one or more variational circuit blocks as described above. The final state may be represented by the following expression:

$$|\psi(n,\theta)\rangle = \prod_{l=0}^{N_l} \left( \prod_{d=0}^{N_d} U_{\mathrm{ent}}^{l,d} U_{\mathrm{sqr}}^{l,d}(\theta_a^{l,d}) U_f^l(n) \right) |0\rangle, \qquad (C3)$$

This final state may subsequently be used in Eq. (13) to define a functional $f_\theta(n) = \langle\psi(n,\theta)|\hat{C}|\psi(n,\theta)\rangle$.

As described above, the XC energy may be computed based on integration of a local functional (eqation (4)) or based on a global functional (equation (5)).

In an embodiment, a quantum model implemented based on a (fully) local QNN (LQNN) $f_\theta^{LQNN}$, may be trained to take a single density point $n_i$ as input and provide an expectation value of a local XC energy density $\epsilon_{XC,i}^\theta$ at its output:

$$f_\theta^{\mathrm{LQNN}} : n_i \rightarrow \epsilon_{\mathrm{XC,i}}^\theta = \langle 0|U^\dagger(n_i,\theta)\hat{C}U(n_i,\theta)|0\rangle, \qquad (14)$$

as defined through Eq. (7). This quantum neural functional may be used in the DFT scheme based on Eq. (4). Local QNNs may include product feature maps and/or Chebyshev feature maps as e.g. described with reference to Eq. C5 and C6 (referred to as local PrQNN and local ChQNN) respectively. An ansatz may be used that has a structure as defined by Eq. (C3) above.

In a further embodiment, the quantum model may be implemented based on a $L$-local QNN,
$$f_\theta^{LQNN} : \{n_i\}^L \rightarrow \epsilon_{XC,i}^\theta = \langle 0|U^\dagger(n,\theta)\hat{C}U(n,\theta)|0\rangle$$ wherein a collection of $L$ neighbouring densities are embedded as input data in gate operation parameters of a feature map.

In another embodiment, the quantum model may be configured as a global QNN. Typically a global model (e.g., $L = N_{grid}$) may provide the best model for approximating exact XC functionals. However this would require a qubit register with $N_{grid}$ number of qubits, which - for a large number of gridpoints - could be a problem for current quantum registers. To broach this problem, in an embodiment, a hybrid neural network including at least a classical neural network and a quantum neural network may be used. Such hybrid neural network may combine advantages of classical NNs and quantum QNNs. Notably, the number of elements $N_{grid}$ in the electron density $n$ scales linearly with the number of atoms. Even for small di-atomic molecules, there are hundreds of grid points for the smallest grid density available with the best integration grids. This may pose an problem when using feature maps based on angle encoding since in that case, the number of qubits $N_q$ required to embed the entire density $n$ equals the size of the grid $N_{grid}$. This poses severe requirements for implementing a global QNN on a near-term quantum computer. Therefore, in an embodiment, a Global QNN (GQNN) may be defined that includes a classical neural network and quantum neural network:

$$f_\theta^{\text{GQNN}}(n) = (f_{\theta_c}^c \circ f_{\theta_q}^q)(n), \qquad (15)$$

where $f_c$ and $f_q$ represent the classical and quantum networks with associated training parameters $\theta_c$ and $\theta_q$, respectively. An example of such hybrid quantum model is shown in Fig. 2B. In principle, any differentiable classical neural network may be used to map outputs of the quantum neural network $f_q$ to an XC energy. Here, the quantum neural network may comprise L-local QNNs defined as

$$f_\theta^q : \{n_i\}^L \to \epsilon_{\text{XC},i} = \langle 0|U^\dagger(n,\theta)\hat{C}U(n,\theta)|0\rangle, \qquad (16)$$

including a feature map block, e.g. a product feature map, and an parametized circuit block, e.g. an alternate ladder ansatz. The input may be partitioned in $N_b = [N_{grid}/L]$ batches of input electron densities and each batch of input electron densities may be embedded in the feature map of the QNN $f_q^\theta$ resulting in $N_b$ outputs which may be used as input of the classical neural network $f_c$ which outputs the exchange energy:

$$f_{\theta_c}^c : \left\{\epsilon_{\text{XC},i}\right\}_{i=1}^{N_b} \to E_{\text{XC}}^\theta, \qquad (17)$$

In an embodiment, the classical neural network may be implemented using a standard MLP with $N_b$ input neurons and one output neuron (potentially with additional MLP network layers). In general, any classical neural network model architecture may be used to represent the classical neural network.

XC functionals may also be designed based on simulated quantum models as quantum-inspired (Qi) solution. The Global QiQNN, which incorporates density through amplitude embedding (following Eq. (C4)) and which may utilize a standard hardware efficient VQE ansatz as described above. The Global QiQNN may be represented by the function

$$F_\theta^{\text{QiQNN}} : \left(\{n\}_{i=1}^{N_{\text{grid}}}\right) \to E_{\text{XC}}^\theta, \qquad (18)$$

wherein an amplitude embedding feature map is used. A global quantum-inspired convolutional NN (GQiCNN) architecture may be used that implements the composition of quantum layers with angle embedding as follows

$$f_\theta^{\text{GQiCNN}}(n) = (f_{\theta_c}^c \circ f_{\theta_{N_l}}^{q,N_l} \circ ... \circ f_{\theta_{q,1}}^{q,1})(n), \qquad (19)$$

where the product feature map (Eq. (C5)) is throughout all the layers.

Similarly to the hybrid architecture (GQNN), the quantum layers may comprise QNNs, for example, L-local QNNs, as defined in Eq. (16). Further, a product feature map and alternate ladder ansatz may be used to define a QNN. The input may be partitioned in $N_b = [N_{grid}/L]$ batches and then $f_q^\theta$ is applied to each batch. This may be repeated $N_l$ times until the output is a single scalar, which represents $E_{XC}$. If not (i.e., odd number of inputs), the output of quantum layers may be combined with an MLP as in Eq. (17) to output $E_{XC}$.

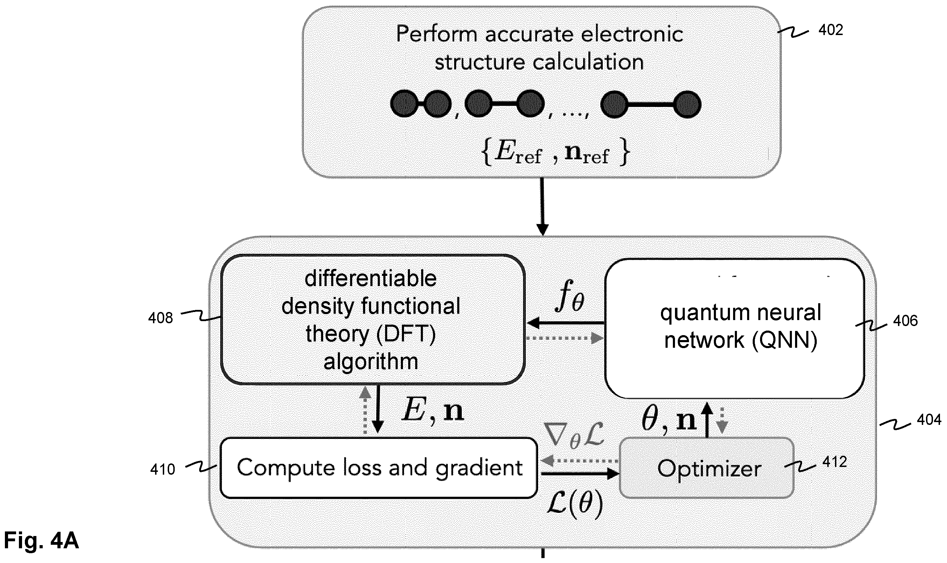

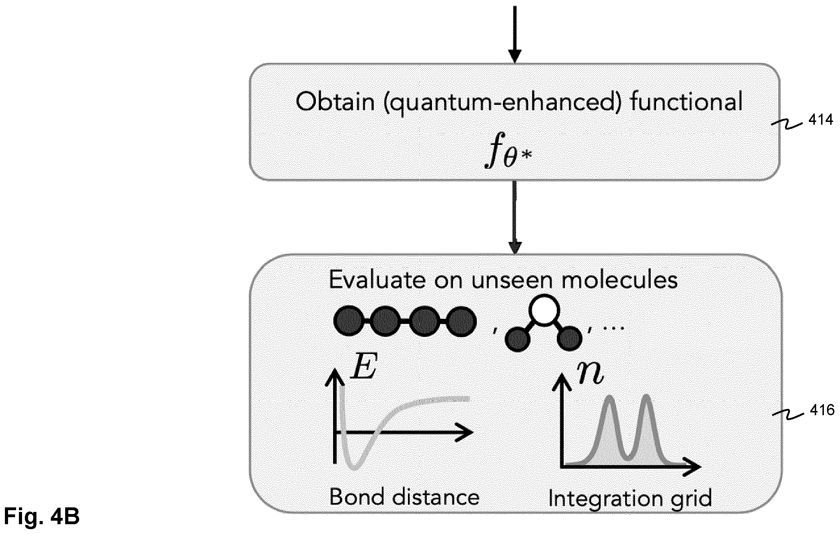

Hereunder, embodiments will be discussed for efficiently training QNN-based XC functions for differentiable chemistry frameworks as for example described with reference to **Fig. 4-6** below.

To evaluate the capabilities of quantum computers for machine learning XC functionals, different differentiable frameworks may be used. Li et al proposed in their article "Kohn-Sham equations as regularizer: Building prior knowledge into machine-learned physics." Physical review letters 126.3 (2021): 036401, a method to facilitate the generalization of neural XC functionals wherein the KS iterative procedure is used as a regularizer. In this method, the energy loss may be computed iteratively at every KS iteration and electron density loss may be computed at the last KS iteration only. At each KS iteration, the loss function is differentiated with respect to the trainable parameters of the QNN. Repeating this process for $N$ KS iterations (solutions of Eq. (1)) enables fast, i.e. in fewer KS iterations, convergence of the quantum model to a reference, while regularizing its parameters.

To implement this method, a fully differentiable chemistry code in 1D in JAX was developed with the aim to demonstrate the regularization capacity of the framework. Since it reduces the number of dimensions (to 1D) and hence simplifies the problem, this is an suitable setup for examining QNN-based models as XC functionals. However, realistic chemistry simulations require a full 3D treatment of molecular systems. To that end, the well-known chemistry framework, PySCF may be used. PySCF has an differentiable version, referred to as PySCF with Automatic Differentiation (PySCFAD). PySCFAD, which is described in the article by Zhang et al, J. Chem. Phys. 157, 204801 (2022), is based on JAX but offers the capability for the accurate treatment of chemical systems in 3D, making it suitable for a wide range of chemical simulations.

In an embodiment, to facilitate the training of neural XC functionals within KS-DFT in 3D, the spin components of the electron density may be constrained to be equal, i.e., $n_\uparrow(r) = n_\downarrow(r)$ and $n(r) = n_\uparrow(r) + n_\downarrow(r)$, thus defining the so-called spin-restricted case of KS-DFT (RKS-DFT). In a further embodiment, the scheme may be extended to the unrestricted case (UKS-DFT), allows the treatment of open-shell systems, which means that one has to transver from a conventional electron density $n(r)$ to a description that is based on an electron density that has two spin components ($n_\uparrow(r)$, $n_\downarrow(r)$) with corresponding potentials $v_{XC,\sigma}[n_\uparrow, n_\downarrow](r) = \delta E_{XC}[n]/\delta n_\sigma(r)$, where spin states are denoted by $\sigma = \{\uparrow, \downarrow\}$. This modification doubles the number of inputs for the neural XC model. Although the examples in this disclosure are based on the RKS-DFT approach, the embodiments are not limited thereto and may also include schemes based other density functional theory algorithms such as UKS-DFT, RKS-DFT or further improvements thereof.

The exact density of an interacting molecular system, which may be computed exactly using a Full Configuration Interaction (FCI) algorithm, allows for fractional occupation numbers, which is preferably for accurate computation of the ground state and accurate training of the XC model. Therefor, in an embodiment, the algorithm of Chai, J. Chem. Phys. 136, 154104 (2012) for fractional occupation may be used to enable fractional occupation numbers. This algorithms provides a computational efficient solution of introducing fractional occupation numbers. Other approaches however may also be employed with an increased computational cost.

The algorithm for fractional occupation as developed by Chai et al. is based on the premisse that the distribution of occupation numbers is independent of the specific many-body system and uses a universal form described by the Fermi-Dirac distribution. In that case, the electron density can be expressed a sum of the electron-orbitals $\psi_i(r)$:

$$n(r) = \sum_{i=1}^{\infty} g_i |\psi_i(r)|^2$$ where the occupation number $g_i$ is the Fermi-Dirac function $g_i = \{1 + exp[[(\varepsilon_i - \mu)/\gamma]]\}^{-1}$ Here,

the occupation number $g_i$ obeys the following two conditions, $\sum_{i=1}^{\infty} g_i = N$ and $0 \leq g_i \leq 1$, where $\varepsilon_i$ is the orbital energy of the $i^{th}$ orbital $\psi_i(r)$ and $\mu$ is the chemical potential chosen to maintain a constant number of electrons $N$. Hence, taken into account the fractional occupation of the electron-orbitals, the expression for the total energy may then be given by

$$E[n] = T_s^\gamma[\{g_i, \psi_i\}] - \frac{\gamma}{k_B} S[\{g_i\}] + \int n(\mathbf{r})v(\mathbf{r})d\mathbf{r} + E_H[n] + E_{XC}^\gamma[n]. \quad (A1)$$

With temperature ($\gamma$) dependent XC energy given by $E_{XC}^\gamma[n] = E_{XC}[n] + E_\gamma[n]$, where $E_\gamma[n]$ represents the difference between the non-interacting kinetic free energy at zero temperature and that at temperature $\gamma$. In that case,

the entropy contribution may be given by $-\frac{\gamma}{k_B} S_s^\gamma[\{g_i\}] = \gamma \sum_{i=1}^{\infty}[g_i \ln(g_i) + (1 - g_i)\ln(1 - g_i)]$. Given a

suitable electronic temperature $\gamma$, the chemical potential $\mu$ is set by $\sum_{i=1}^{\infty}\{1 + exp[[(\epsilon_i - \mu)/\gamma]]\}^{-1} = N$ to conserve the number of particles. Hence, in an embodiment, in order to take account of fractional occupation numbers, only the entropy contribution needs to be added to an existing DFT implementation so that $E_{XC}^\gamma[n]$ can be learned by the QNN. This approach is particular advantageous as it does not impact the overall computational scaling. The improved density representability requires the inclusion of a single free parameter, the temperature $\gamma$ as described above.

**FIG. 4A** and **4B** depict a process for computing electronic properties of a molecular system using a hybrid computing system according to an embodiment.

As shown in **Fig. 4A,** the process may include providing and/or computing a dataset **402** for training a quantum neural network (QNN). The training dataset may comprise reference data, in particular reference electron-orbital features such as reference electronic densities $n_{ref}$ and associated ground state energies of atomic and/or molecular systems. The reference data include synthetic data reference data which may be computed using accurate ab initio methods such as full configurations interation (FCI) calculations. Alternatively and/or in addition, the reference data may include experimental data, regarding electron-orbital features such as electron densities and ground state energies for the different atomic and/or molecular systems.

These data may be obtained by techniques that can be used to probe electronic properties of atomic and molecular systems. For example information about the relative position of atoms in molecular systems may be obtained using X-ray crystallography and information about electron densities may be obtained by (but are not limited to) techniques that atomic force microscopy (AFM), scanning tunnelling microscopy (STM) or transmission electron microscopy (TEM) and spectroscopy techniques such as angle-resolved photoelectron spectroscopy. Based on these data, information about the quantum mechanical wave function of an atomic or molecular system can be obtained.

Then, training process **404** may be executed, wherein a differentiable quantum neural network (QNN) **406** is trained based on the reference data. In particular, the training process may be used to train the trainable parameters $\theta$ of the QNN based on the reference data of the different atomic and/or molecular systems.

The training scheme may include selecting an initial (first) electron density $n$ of a atomic or molecular system for which reference data are available. Then, a new (second) electron density and associated ground state energy may be computed based on the initial (first) electron density $n$ and based on a differentiable density functional algorithm **408** and an XC energy computed by a trainable QNN associated with first trainable parameters $\theta_1$ using the first electron density as input data. In an embodiment, the differentiable density functional algorithm may be a Density Functional Theory (DFT) algorithm such as the KS-DFT algorithm.

The computation of the new (second) electron density and the associated ground state energy may be computed using an self-consistent density functional algorithm, such as the KS-DFT algorithm. This computation may include determining an estimate of an XC energy which is generated by the differentiable QNN using the initial (first) electron density as input data. A corresponding XC potential $v_{XC}$ may be computed by differentiation of the computed XC energy with respect to the electron density: $v_{XC} = \delta E_{XC}/\delta n(\boldsymbol{r})$. The XC potential may then be used by the differentiable density functional algorithm to compute new electron-orbitals that can be used to determine the new (second) electron density. For example, Fock equations of the KS-DFT algorithm may be used to compute new orbitals from which the new (second) density may be determined. If the difference between the newly computed electron and the earlier (initial) electron density is lager than a certain delta, then the new (second) electron density for computing a new electron density and associated ground state energy. If the difference between the newly computed electron and the earlier (initial) electron density is smaller than a certain delta, the computed electron density and associated ground state energy may be used for computing a loss.

In particular, a loss function $\mathcal{L}(\theta)$ **410** may be used to evaluate the computed density and the associated ground state energy using the reference data, for example a reference electron density and a reference ground state energy of the selected atomic or molecular system. Based on the computed loss, it may be determined to update the trainable parameters of the QNN using a classical optimizer **412** wherein the updating of the parameters may include determining a gradient of the loss with respect to the trainable parameters $\nabla_\theta \mathcal{L}$. Hence, after the update of the parameters, the QNN is associated with second trainable parameters $\theta_2$.

Subsequently, the self-consistently computed electron density and the associated ground state energy may be used as an input to the self-consistent density functional algorithm for computing a further energy density and associated groundstate. The training process **404** may be repeated for the different atomic and/or molecular systems for which reference data **402** are available.

Fig. **4B** further illustrates the use of the trained QNN as an XC functional. If convergence of the trainable parameters have been reached , the converged parameters $\theta^*$ may define the trained functional $f_{\theta^*}$ **418.** i.e. the trained QNN representing the XC functional. This trained QNN representing a quantum-enhanced XC functional, may then be used to evaluate electronic properties, such as the ground state energy or the electron density of a new atomic or molecular system (step **420).** These electronic properties may be computed based on a density functional computation cycle as described with reference to **Fig. 4A,** using the trained QNN presenting the functional $f_{\theta^*}$ to compute the XC energy. The thus obtained function provides improved performance for computing or predicting electronic properties of atomic or molecular systems, which is particular relevant in quantum chemistry in which the computed or predicted properties asre used in determining a decision about a chemistry process in the real-word, e.g. fabricating or synthesying a particular new molecular system based.

For example, the process may be used to compute the ground state energy for a new molecular system or for an intermediate molecular systems that are part of a chemical synthesis scheme. The ground state energy can be used to determine if a (intermediate) molecular system is stable or if atoms in a new molecular system can bind.

In an embodimenmt, a supervised ML approach may be used to train a QNN to represent an XC functional. A

training dataset $D = \{E_{ref}^i, n_{ref}^i\}_{i=1}^{N_{mol}}$ (also referred to as a refenrece dataset) may be generated using an accurate *ab initio* method (for example FCI) for $N_{mol}$ different moleculair systems (which may also include different geometries of same molecules). Alternatively and/or in addition the training data set may include experimentally determined data. In an embodimemt, in case of a 1D implementation, the training data may comprise training data as determined by Li et al. "Kohn-Sham equations as regularizer: Building prior knowledge into machine-learned physics." Physical review letters 126.3 (2021): 036401. This dataset may include reference data for hydrogen-based molecules ($H_2$, $H_2^+$ and $H_4$). For a 3D implementation, computational schemes such as Python-based Simulations of Chemistry Framework (PySCF) and/or Coupled Cluster Singles Doubles (CCSD) may be used to generate data for hydrogen type molecular system.

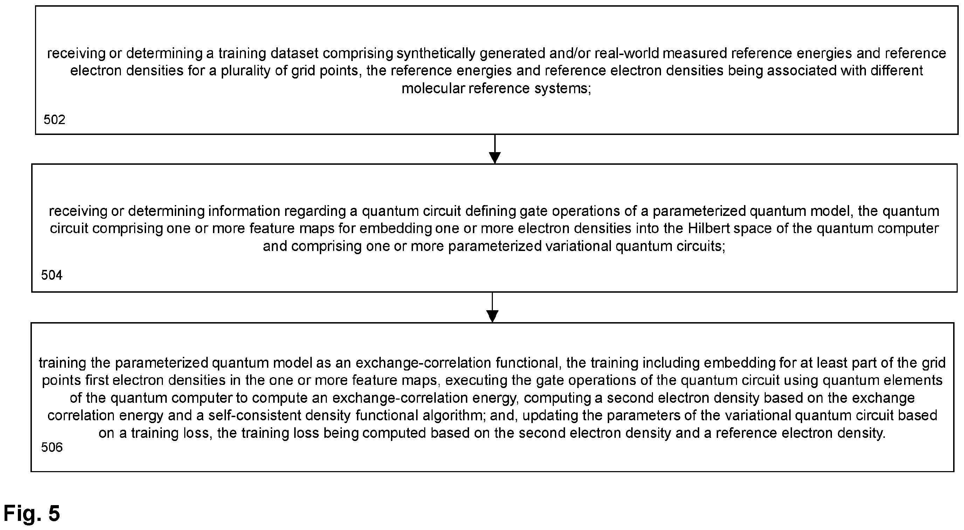

**Fig. 5** depicts a flow chart for training a quantum model as an exchange-correlation functional according to an embodiment. In particular, the figure depicts a method of training a quantum model, e.g. a QNN, as an XC functional as e.g. described with reference to **Fig. 4,** wherein, in a first step **502,** the method may include receiving or determining a training dataset comprising synthetically generated and/or real-world measured reference electron densities and reference energies for a plurality of grid points, the reference electron densities and reference energies being associated with different molecular reference systems.

In a further step **504,** the method may include receiving or determining information regarding a quantum circuit defining gate operations of a parameterized quantum model, wherein the quantum circuit may comprise one or more feature maps for embedding an electron density into the Hilbert space of the quantum computer. The quantum circuit may further comprise one or more parameterized variational quantum circuits having trainable parameters may be used to act on the embedded data using local qubit operations and entangling gates.

Then, the parameterized quantum model may be trained as an exchange-correlation functional (step **506**), wherein the training may include embedding for at least part of the grid points first electron density in the one or more feature maps. The gate operations of the quantum circuit may be executed using quantum elements of the quantum computer in order to compute an exchange-correlation energy. Further, a second electron density may be computed based on the exchange correlation energy and a self-consistent density functional algorithm, such as a DFT algorithm. Then, the parameters of the variational quantum circuit may be updated based on a training loss, wherein the training loss may be computed based on the second electron density and a reference electron density.

Hence, the method provides an efficient way of training a QNN as a XC functional that is used in a density functional theory scheme for computing electronic properties of a molecular system.

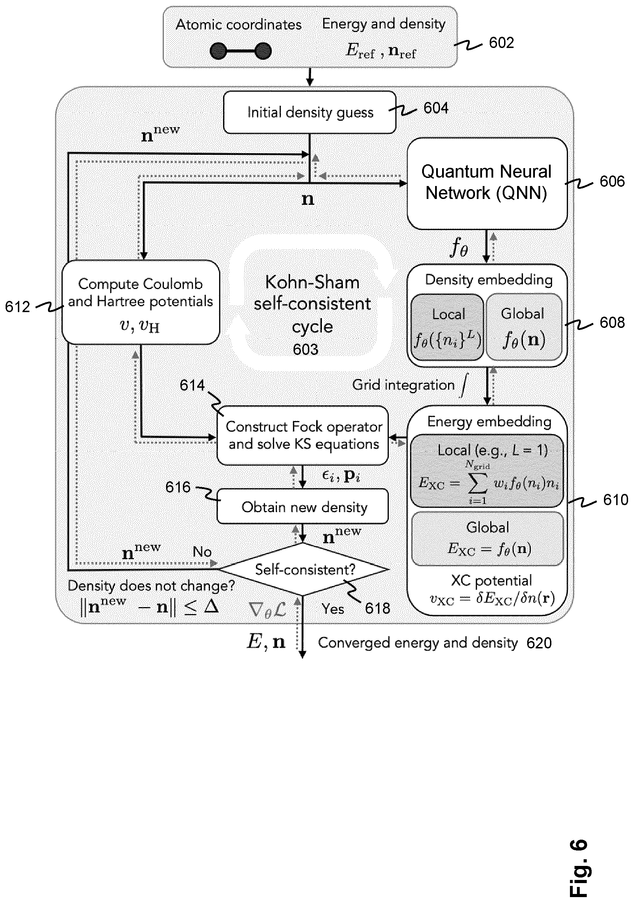

**Fig. 6** depicts the process of computing an electron density based on a self-consistent density functional algorithm according to an embodiment. In particular, the figure depicts a process of computing an electron density based on a self-consistent density functional algorithm as described in **Fig. 4A** in greater detail.

The process may start with providing or determining training data of a plurality of different moleculair systems (step **602**), wherein the training data may include reference electronic-orbital features, such as reference energy and reference electron density of these different molecutlar systems. The training data may further include atomic coordinates forming a discrete grid of $N_{grid}$ points in space to which electron-orbital features may be assigned. In a next step **604,** an initial electron density may be estimated (e.g. using a classical computation and/or using experimental data) which can be used as input to a density functional algorithm for computing an estimate of the energy density of a molecular system and the associated ground state energy. In particular, the initial electron density $n$ estimate may be used to compute the Coulomb and Hartree potentials $v, v_H$ (step **512**). Further, the electron density $n$ estimate may be provided to the input of a QNN, including one or more feature maps and one or more variational circuits, which is trained as a XC correlation function $f_\theta$ (step **606**) to compute an XC energy. Then, the electron density may be embedded in the QNN (step **608**), different ways of embedding the electron density values in the QNN. In an embodiment, the QNN may be configured to receive $L$ density values as inputs wherein $1 \leq L \leq N_{grid}$. In case of $L = 1$ (i.e. one point of the integration grid) the electron density is fully locally embedded in the gate parameters of the feature map. In case of $L = N_{grid}$ (i.e. the full integration grid) the embedding of the input data is globally embedded in the gate parameters of the feature map. Other values of $L$ may be used for the embedding.

After the embedding of the input data, the gate operations of the quantum circuit defining the QNN may be executed on the quantum computer to compute the XC energy (step **610**). In case of an QNN with local embedding, the output of the QNN will be an energy value $\varepsilon_i$ for a particular electron density value $n_i$ on a point of the integration grid. Hence, in that case, the QNN needs to be executed multiple times so that the sum of all computed values at all grid points of the integration grid will result in the XC energy. An QNN with global embedding will output an energy value representing the total XC energy for the whole integration grid. Thereafter, the XC potential $v_{XC}$ may be computed as the derivative of the XC energy with respect to the energy density (step **611**). Based on the computed Coulomb and Hartree potentials $v, v_H$, the computed XC potential $v_{XC}$ and the computed energies $\varepsilon_i$ at the grid points, a Fock operator may be constructed and the KS equations may be solved to obtain electron-orbital coefficients $p_i$ (step **614**). These electron-orbital coefficients may be used to compute a new density value $n^{new}$ (step **616**), which may be compared with the earlier electron value (step **618**).

If the difference between the old and new density value is larger than a certain delta value $\Delta$, the newly computed electron density value may be used as the new input value (step **613**) to start a next computation cycle (i.e. steps **606-618**) until the difference between subsequently computed electron densities is smaller than the delta value. If that condition is met, the self-consistency is reached and the density function algorithm may be stop. The computed estimated energy and electron density associated with a molecular system (step **620**) may then be used in a training process as explained with reference to **Fig. 4A,** wherein the variational parameters of the QNN may be updated based on a loss function, the computed electron density and associate ground state energy and the associated reference data from the training data.

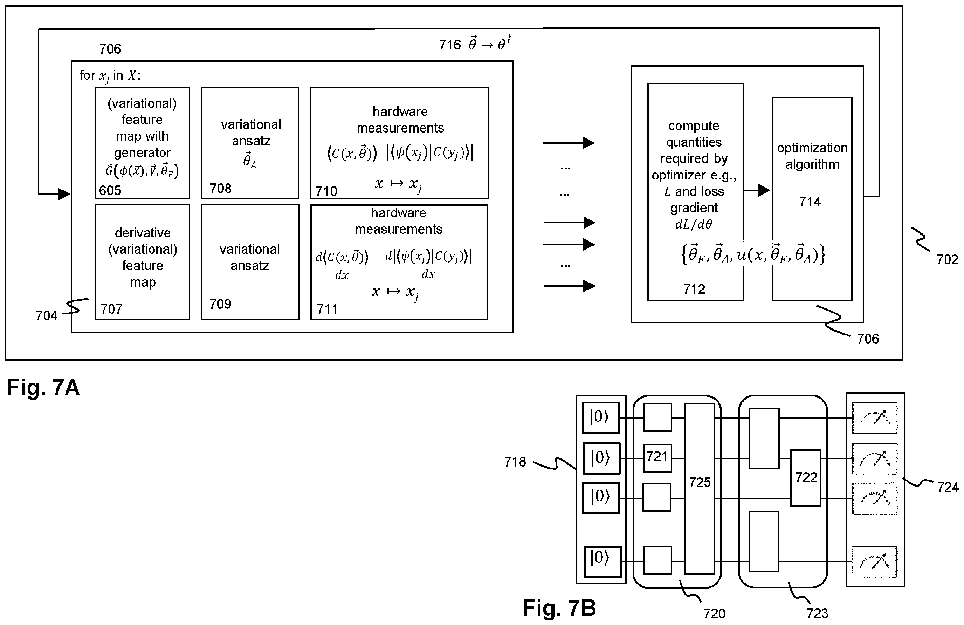

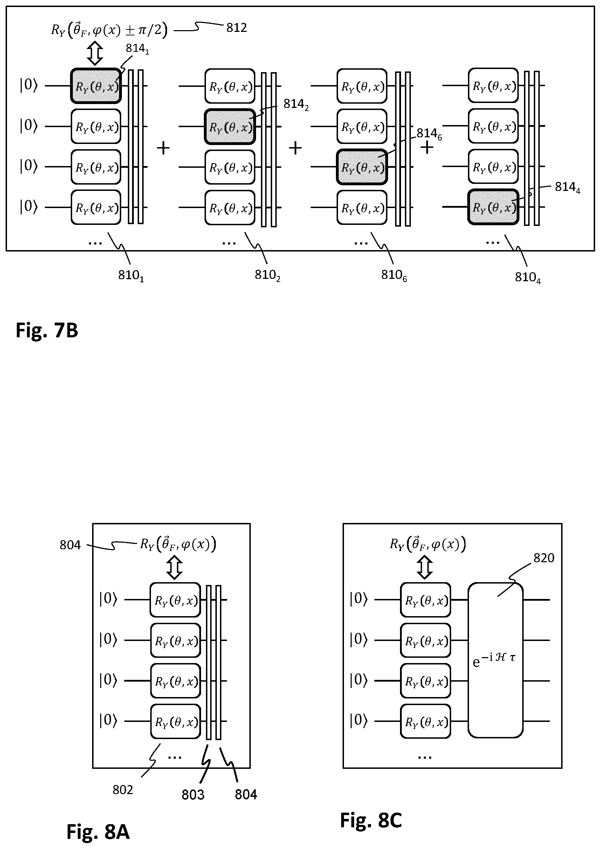

**Fig. 7A** and **7B** depict a scheme for executing a QML model that may be used for solving a density functional theory problem in molecular chemistry as described with reference to the embodiments in this disclosure.

In an embodiment, a QML model may be on differentiable quantum circuits (DQC) may be used. DQC schemes are described in WO2022/101483 with title *Solving a set of (non)linear differential equaitons using a hybrid data processing system comprising a classical computer and a quantum computer system,* which is hereby incorporated by reference into this application. In the DQC scheme, the QML may include a quantum circuits for modelling a surrogate solution $f(x)$ e.g. an XC functional that is configured to receive input data $x$, e.g. electron-orbital features such as electron densities as described with reference to the embodiments in this application.

The quantum circuit may include one or more feature maps and one or more variational quantum circuits. In an embodiment, only the variational quantum circuit may be parameterized for training. In another embodiment, both the one or more features maps and the one or more variational quantum circuits may be parameterized for training. In particular, in an embodiment, the quantum circuit representing the QML model may include one or more variational feature maps $\hat{U}(\vec{x}, \vec{\theta}_F)$ **705** with a parameterized generator Hamiltonian $\hat{G}(\phi(\vec{x}),\vec{\gamma},\vec{\theta}_F)$ and one or more parameterized ansatze $\hat{U}_A(\vec{\theta}_A)$ **708** for computing function values $f(x_i)$ for different input values of $x_i$, e.g. electron densities. Hence, in this case the feature map may also include variational parameters which may be updated when training the QNN. In a further embodiment, QML models based comprising one or more trainable feature maps are described in EP application no. 23186558.5 with title *Solving computational problems using trainable feature maps* which is hereby incorporated by reference into this application.

In some embodiments, the quantum circuits may also include derivative quantum circuits **707** which may be determined using auto-differentation and the parameter-shift rule. The derivative quantum circuits **707** and associated variational quantum circuits **709** may be used to determine a value of a function derivative $\left.\dfrac{df}{dx}\right|_{x_i}$ for different values of $x_i$. As shown in **Fig. 7B,** the quantum circuits may be executed on a quantum computer comprising a quantum register **718** comprising quantum elements, such as qubits or qudits. The quantum circuits including one or more variational features maps **720** and variational ansatze **723** may comprise a sequence of single and/or multiple operations **721,722,725** that are applied to the quantum elements, followed by a read-out operation **724** to read-out the state of the qubit register. When executing the quantum circuits, the operations are translated into a sequences of pulses tho control the quantum elements and readout the quantum elements.

The quantum circuits may be executed based on an optimization schedule, wherein the execution may include execution of the quantum circuits and measuring the state of the quantum regiter **704.** A set of points $\{X\}$ (a regular or a randomly-drawn grid) may be specified for each equation variable $x$ **706**. Variational parameters $\vec{\theta}_F, \vec{\theta}_A$ may be set to initial values (e.g. as random angles or a predetermined set of intial anges) and the operations may be executed by the quantum register as shown in **Fig. 7B** and the state of the quantum register may be measured.

Using a cost function $\hat{C}$, expectation values $\langle C(x, \vec{\theta}_F, \vec{0}A)\rangle$ and $\dfrac{d\langle C(x,\vec{\theta}_F,\vec{\theta}_A)\rangle}{dx}$ with respect to the variational quantum state $|\psi_{\theta_A,\theta_F}(x_i)\rangle$ can be determined (steps **710-711**), for the chosen point $x_j$ to represent the surrogate solution at that point $f(x_j)$.

In another embodiment, using a cost quantum wavefunction $|C(y_j)\rangle$ the overlaps $|\langle\psi(x_j)|C(y_j)\rangle|$ and $\dfrac{d|\langle\psi(x_j)|C(y_j)\rangle|}{dx}$ can be determined (steps **710-711**), for the chosen point $x_j$ to represent the surrogate solution. The cost wave function can be parameterized by the encoding of a second point $y_j$ and additional action of other trainable parameters, or a fixed reference state (e.g., $|C(y_j)\rangle = |000 ...\rangle$, the wavefunction where all qubits are in the computational state $|0\rangle$). In both embodiments, a potential solution at this point may be constructed taking into account the boundary conditions.

Further, the expectation values $d\langle C(x, \vec{\theta}_F, \overline{\theta}_A)\rangle/dx$ associated with the derivative quantum circuits may be estimated **711** for a predetermined cost function at point $x_j$. Repeating the procedure **706** for all points $x_j$ in $\{X\}$, function values and derivative values may be computed. Further a loss function and other quantities required by the optimizer **712** may be determined (forming required polynomials and cross-terms by classical post-processing). In some embodiments, regularization points may be added, forcing the solution to take specific values at these points. The loss function may be used to determine a loss value, which may be used to determine a score $u$ (a metric) which is indicative to how well the QNN (parametrized by variational angles $\vec{\theta}_F$, $\vec{\theta}_A$) is capable of computing the XC energy, matching derivative terms and the function polynomial to minimize the loss.

With the aim to increase the score (and decrease the loss function), the gradient of the loss function **712** with respect to variational parameters $\vec{\theta}_F$, $\vec{\theta}_A$ may be computed. Using classical optimization procedure **714,** such as the gradient descent procedure, the variational parameters may be updated from iteration $n_j = 1$ into the next one $n_j + 1$ in step

**716**: $\theta^{(n_j)} \leftarrow \theta^{(n_j+1)} - \alpha \nabla_\theta \mathcal{L}$ (with $\alpha$ being here a 'learning' rate). The above-described steps may be repeated until one or more exit conditions are reached. Suitable exit condition may include:

1) the maximal number of iterations $n_{iter}$ reached;
2) loss function value is smaller than pre-specified value; and
3) loss gradient is smaller than a certain value.

Once the classical loop is exited, the solution may be chosen as a circuit with angles $\vec{\theta}_{F_{opt}}$, $\vec{\theta}_{A_{opt}}$ that minimize the loss. Finally, the full solution may be extracted by sampling the cost function for optimal angles

$$\left\langle u_{\varphi,\vec{\theta}_{F_{opt}},\vec{\theta}_{A_{opt}}}(x) \middle| \hat{\mathcal{C}} \middle| u_{\varphi,\vec{\theta}_{F_{opt}},\vec{\theta}_{A_{opt}}}(x) \right\rangle.$$

Notably, this can be done for any point $x$, as DQC constructs the solution valid also beyond (and between) the points at which loss is evaluated originally.

In a further embodiment, XC functionals may also be evaluated by evaluating quantum kernels and derivatives thereof. A QML model based on differential quantum kernels may be used. Such differential quantum kernel schemes are described in WO2023170003 with title *"Quantum kernel based regression",* which is hereby incorporated by reference into this application. In that case, kernel values $\kappa(x_j)$ and a kernel derivative values $\nabla_m^n \kappa(x_j)$ may be computed for different input values $x_j$. These quantum feature maps, which may be referred to quantum kernel feature maps, can comprise unitary operations that are similar to unitary operations of the trainable features map described with reference to the embodiments in this application. Application of operations on the quantum register will results in a variational quantum state $|\psi_{\theta_F}(x_j)\rangle$. Subsequently, in an embodiment, kernel values may be determined as a wavefunction overlap of the a variational quantum state and another reference state $|\langle\psi_{\theta_F}(x_j)|\psi_r\rangle|$. Note that in other parts of this document and figures, the reference state $|\psi_r\rangle$ is also referred to as a cost state $|\hat{C}\rangle$, which may arbitrarily depend on another input point or parameterization such that it is written as $|C(y_j)\rangle$. A kernel value $\kappa$ may be measured using a well-known Hademard test configured to measure the real and imaginary part of the overlap:

$$\kappa(x) = \left\langle \psi_{\theta_F}(x_j) \middle| \psi_r \right\rangle = \mathrm{Re}\left(\left\langle \psi_{\theta_F}(x_j) \middle| \psi_r \right\rangle\right) + \mathrm{Im}\left(\left\langle \psi_{\theta_F}(x_j) \middle| \psi_r \right\rangle\right)$$

Similarly, derivatives of the kernel function may be be computed on the quantum computer using similar operation sequences resulting in a measurement of a kernel derivative value. The measurement of a kernel value typically involves repeated measurements on the same circuit, the quantity of interest being an average of the repeated measurements.

Thus, from the above, it follows that a broad class of feature maps can be described as the tensor product of an arbitrary number of sub-feature-maps, each represented by the time evolution of a generator Hamiltonian applied on an arbitrary sub-dimension of the qubits

$$\hat{U}_F(\vec{x}, \vec{\gamma}) = \bigotimes_m e^{-i\hat{G}_m(\vec{\gamma})\phi_m(\vec{x})},$$

where for the sub-feature-map m, $\hat{G}_m(\vec{\gamma})$ is the generator Hamiltonian that depends on non-trainable parameters $\vec{\gamma}$ and $\phi_m(\vec{x})$ is an encoding function that depends on the input features $\vec{x}$. Note that $\hat{U}_F(\vec{x})$ can be applied several times across the quantum circuit, interleaved with $\hat{U}_A(\vec{\theta}_A)$ layers, for example in data re-uploading or serial feature maps. In data re-uploading, $\hat{U}_F(\vec{x})$ is applied more than once in non-parallel blocks separated by other quantum operations which could be $\hat{U}_A(\vec{\theta}_A)$, so that the data is "re-uploaded" into a different qubit basis. This is understood to increase the number of unique frequencies of the model. In serial feature maps, multi-dimensional data may be encoded by non-parallel blocks that are separated by other quantum operations which each encode one dimension e.g., $\hat{U}_F(x_1)\hat{U}_A(\vec{\theta}_A)\hat{U}_F(x_2)\hat{U}_A(\vec{\theta}_A)$.

Introducing trainable parameters in the generator Hamiltonian $\hat{G}_m(\vec{\gamma}, \vec{\theta}_F)$ of the quantum model, produces a measured output which can be expressed as a Fourier series, where the frequencies of each mode depend explicitly on the parameterization of the FM. For example, for a QNN with a cost $\hat{C}$, the output of the model can be written as:

$$f(\vec{x}, \vec{\theta}_A, \vec{\theta}_F) = \sum_{\omega_j \in \Omega} c_j(\vec{\theta}_A, \hat{C}) e^{i\omega_j(\vec{\theta}_F)\phi(\vec{x})},$$

Where $\Omega$ contains the spectral gaps of all generators and $c_j$ are the corresponding coefficients. Thus, introducing trainable parameters into the generator, which allows the eigenvalue spectrum to change over the course of training, leads to a quantum model with trainable eigenfrequencies $\omega_j(\vec{\theta}_F)$. This - in turn - allows the quantum model to "discover" a choice of spectral modes not known a priori that may minimize the loss function more efficiently than non-trainable FMs. Since the number of frequencies accessible to the model $|\Omega|$ scales with the Hilbert space dimension, parameterization of each frequency individually would require exponentially many parameters. The effectiveness of a simple parameterization that grows only linearly with the number of qubits is described with reference the example below. The advantage of trainable feature maps (TFMs) stems from two important technical effects. Firstly, TFMs are able to be trained such that their frequency spectra contain non-uniform gaps, producing non-orthogonal basis functions.

**Fig. 8A-8C** depict quantum circuits which may be used in the embodiments in this disclosure. **Fig. 8A** shows a basic form of a quantum feature map, which is here illustrated as an example of a 'product' type feature map. In this example, single qubit rotations **804** (here chosen as $R_Y(\varphi(\boldsymbol{x}))$) act on each qubit individually and are parametrized by a function $\varphi$ of variable $\boldsymbol{x}$. The function $\varphi$ is preferably a non-linear function. For a non-linear feature map encoding, the non-linear function $\varphi(x)$ may be used as an angle of rotation. The application **802** of one rotation operation to each qubit in a register may be referred to as a single layer of rotation operations. A product feature map can be further generalized to several product layers, and different functions $\{\varphi\}$; for example, several feature maps may be concatenated to represent a multivariable function. For a quantum kernel, a single layer (as in **Fig. 8A**) or a double layer (as in Fig. 8B) is generally sufficient.

Fig. 8B illustrates an example of a derivative quantum circuit for the product feature map of **Fig. 7A.** Differentiation over variable $x$ follows the chain rule of differentiation, including qubit operations **812** with shifted phases $R_Y(\varphi(\boldsymbol{x}) \pm \pi/2)$. Here, the expectation value of the derivative is written as a sum of separate expectations with shifted phases, repeated for each $x$-dependent rotation **810$_{1\text{-}4}$**.

**Fig. 8C** depicts an example of a generalized product feature map, where the layer of rotations is followed by the unitary evolution generated by Hamiltonian $\mathcal{H}$ **820**. For complicated multiqubit Hamiltonians, the encoded state may comprise exponentially many unique $x$-dependent amplitudes. The time interval $\tau$ can be set variationally, or annealed from zero to a finite value during the optimization procedure.

Preferably, the product feature map has a non-linear dependence on the encoded variable $x$. In the simplest case, this may correspond to a single layer of rotations as shown in **Fig. 8A.** Such product feature map may be described by the following expression:

$$\mathcal{U}_\varphi(x) = \bigotimes_{j=1}^{N'} R_{a,j}(\varphi(\boldsymbol{x})) \ ,$$

where $N' \leq N$ is a number of qubits that is used by the quantum computer for the encoding and the symbol $\otimes_j$ denotes the tensor product. Further, $R_{a,j}(\varphi) = \exp\left(-i\frac{\varphi}{2}P_{a,j}\right)$ is a Pauli rotation operator for Pauli matrices $\hat{P}_{a,j} = \hat{X}_j, \ \hat{Y}_j \ or \ \hat{Z}_j$, ($a = X, Y, Z$, respectively) acting at qubit $j$ for phase $\varphi$. This type of feature map circuit may also be used in Quantum Circuit Learning (QML).

The next step is to assign a non-linear function for rotation. In an embodiment, the non-linear function may be selected as $\varphi(\boldsymbol{x}) = \arcsin(\boldsymbol{x})$ and $a = Y$, such that only real amplitudes are generated. The unitary operator may then be rewritten as (for $N' = N$):

$$\mathcal{U}_\varphi(\boldsymbol{x}) = \bigotimes_{j=1}^{N} \exp\left(-i\frac{\arcsin(\boldsymbol{x})}{2}\hat{Y}_j\right),$$

leading to amplitudes that depend on the encoded variables as sin(arcsin($\boldsymbol{x}$) /2) and cos(arcsin($x$) /2). Acting on the initial state $|0\rangle$, this feature map may encode the variable as an N-th degree polynomial formed by $\{1, x, \sqrt{1 - x^2}\}$ and products thereof.

The product feature map can be generalized to several layers of rotations $\ell$ = 1, 2, ..., $L$, various non-linear functions $\varphi_\ell$ and specific subsets of qubits $N$ written as:

$$\mathcal{U}_\varphi(\boldsymbol{x}) = \prod_{\ell=1}^{L} \bigotimes_{j \in \mathbb{N}_\ell} R^{(\ell)}{}_{a,j}(\varphi_\ell(\boldsymbol{x})).$$

In a further embodiment, a quantum model based on a parallel DQC scheme may be used. Methods and systems based on the parallel DQC scheme are described in EP application no. 23186602.1 with title *Quantum circuits,* which is hereby incorporated by reference into this application.

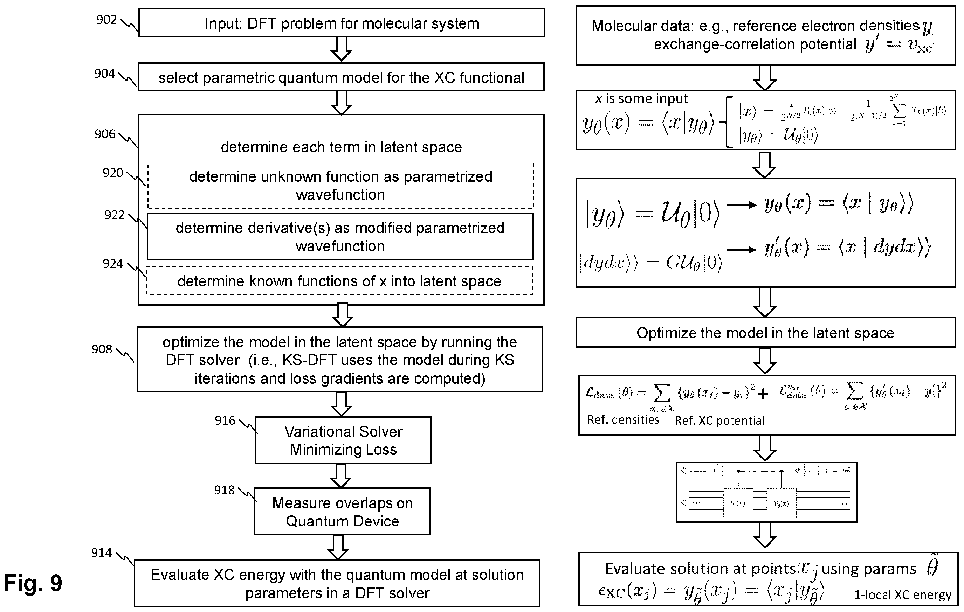

**Fig. 9** depicts the use of another scheme for executing an QML model that may be used for solving a density functional theory problem in molecular chemistry as described with reference to the embodiments in this disclosure. In this DQC scheme a Chebyshev or Fourier feature map may be used.

In particular, in this embodiment, the quantum model for XC functional may be represented as a projection: $\varepsilon_{XC}(x)$ = $y_\theta(x) = \langle x | y_\theta \rangle$ where the $|x\rangle$ represents the input data (e.g., electron density) that is mapped via Chebyshev or Fourier embedding in the Hilbert space, and $|y_\theta\rangle = U(\theta)|0\rangle$ the quantum state after application of a parametrized ansatz $U(\theta)$ on an arbitrary initial state 10). The gradients with respect to the input data x then may be computed via the projection $y'_\theta(x) = \langle x | G | y_\theta \rangle$ where $G$ represents a non-unitary differentiation operator. This model may be referred to as the parallel DQC model. To train the model, a mean squared error loss may be used with respect to the reference data $y_i$ (e.g., CCSD(T) electron densities) as

$$L_{\text{data}} = \sum_i (y_\theta(x_i) - y_i)^2 .$$

In a first step **902,** information regarding a density function theory problem in molecular chemistry may be provided. As shown in the figure this step may include providing molecular data, e.g. reference electron densities and/or energies associated with different molecular systems. Further, (step **904**) a parametric quantum model based on the parallel DQC model may be selected wherein each term of a XC functional may be written as one or more terms in a latent space. This may comprise a step **920,** comprising representing the unknown XC functional as a parametrized wavefunction, a step **922** comprising determining one or more (first-order and/or higher-order) derivatives of the unknown XC functional with respect to the independent variable as modified parametrized wavefunctions, and - optionally - a step **924** comprising determining one or more known functions as wavefunctions. A subsequent step (not shown) may comprise receiving or determining one or more quantum circuits defining operations of the parameterized quantum model, wherein the operations effectuate the encoding determined in the previous step. Different quantum circuits defining operations of the parameterized quantum model may be found in EP application no. 23186602.1.

Then, the XC functional may be determined by optimizing the model in the latent space by running a DFT scheme, wherein DFT scheme uses the model during KS iterations and computation of loss gradients. The optimization of the model may include a variational solver **916** and a step of measuring overlaps on a quantum processor **918.**

After optimization of the model, the DFT scheme (DFT solver) and the optimized quantum model may be used to evaluate electronic densities and energies of a new (unknown) molecular system that differs from the reference molecular system that were used during optimization (training) of the quantum model.

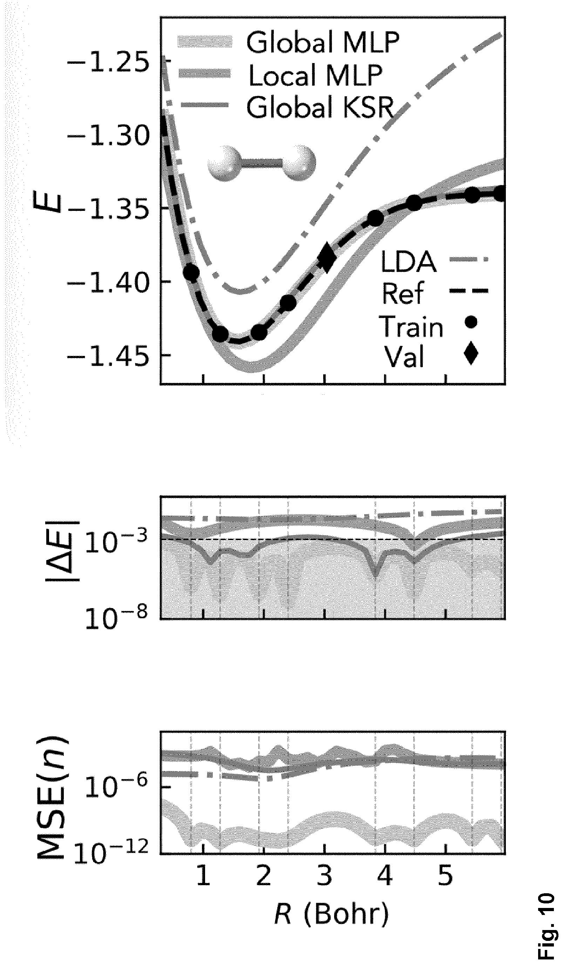

**Fig. 10** depicts experimental data regarding computation of electronic states of a molecular system using a density functional algorithm based on a classical and quantum-enhanced XC functionals. The experimental data are computed based on a classical MPL-type neural network (as described with reference to equations (10)-(12), which may be used as a reference for comparing the experimental data associated with the quantum models,

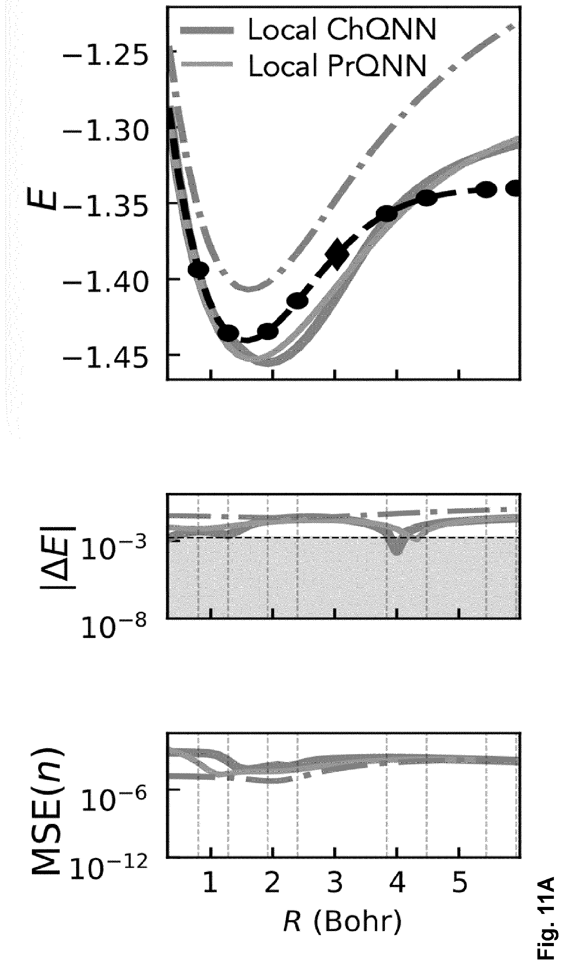

**Fig. 11A-11C** depict experimental data regarding computation of electronic states of a molecular system using a density functional algorithm based on quantum-enhanced XC functionals. In particular, Fig. 11A-C depict experimental data computed based on a fully quantum model, a hybrid quantum model or a quantum-inspired model as described with referencs to embodiments in this application. The experimental data in the figures illustrate the of training neural XC functionals on different geometries of hydrogen molecule in 1D KS-DFT framework of Li et al.. Local models improve but qualitative follow the LDA shape. Best results are obtained for global models when the whole electron density is considered as model input. The reference data (ref) depicted in the figures are generated using FCI. LDA results are shown for comparison with local quantum (hybrid) models. All neural functionals are trained on eight training data points (circles) and the final model parameters are selected based on best loss on the validation results. The model architectures that were use to compute these data are described with reference to equations (6)-(19) above.

When more than two electrons are present, the CCSD with approximate Triples (CCSD[T]) method is used, which is the "golden standard" in computational chemistry. This method is described in detail in the article by R. J. Bartlett and M. Musial, Reviews of Modern Physics79, 291 (2007). Gaussian-type orbital basis set is used all systems, in particular 6-31G. As integration grid, SG-3 with Becke scheme may be used to combine multi-atomic grids, such that for a given molecule at all geometries the same number of grid points are obtained. The grid density may be set to minimum to reduce the number of neural XC functional inputs, thereby reducing the complexity of the problem. Given that models are trained that allow a fixed number of inputs ($N_{grid}$), grids have to be adapted (e.g., pruning) to have the same number of grid points for all molecules in the dataset. However, this is only a limitation imposed by simple prototypical (quantum) models that are investigated in this work.

To train the models, a loss function may be used given by

$$\mathcal{L}(\theta) = \underbrace{\frac{1}{N_{\text{mol}}} \sum_{i=1}^{N_{\text{mol}}} \left[ \sum_{j=1}^{N_{\text{grid}}} w_j \left( n_{\text{KS,j}}^i(\theta) - n_{\text{ref},j}^i \right)^2 / N_e^i \right]}_{\text{density loss } \mathcal{L}_n}$$

$$+ \underbrace{\frac{1}{N_{\text{mol}}} \sum_{i=1}^{N_{\text{mol}}} \left[ \sum_{k=1}^{N_{\text{KS}}} \eta_k \left( E_k^i(\theta) - E_{\text{ref}}^i \right)^2 / N_e^i \right]}_{\text{energy loss } \mathcal{L}_E},$$

$$(21)$$

where the expectation is taken on $N_{mol}$ molecules of the training set and $\eta_k$ weights the contribution of every energy at each KS iteration. Hence, generally, in an embodiment, the loss function may include a density loss $\mathcal{L}_n$ computed based on the predicted electron densities (associated with parameter $\theta$) and the reference densities at each point of the grid and an energy loss $\mathcal{L}_E$ computed based on the predicted energies (associated with parameter $\theta$) and the reference energies at each point of the grid.

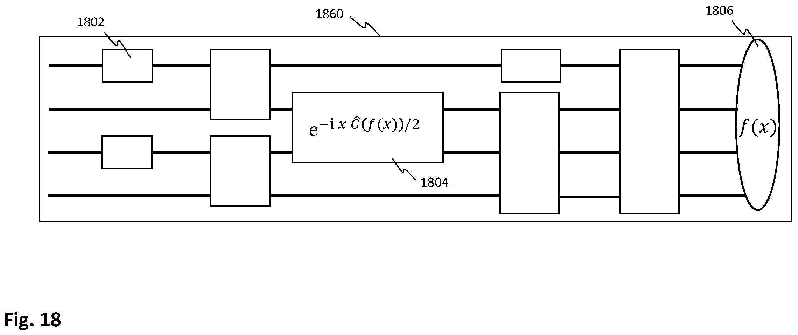

For the energy loss a weighting scheme as described by Li et al. may be used $\eta_k = 0.9^{NKS-k}H(k-10)$, where $H$ is the Heaviside function to tune the contribution of every loss term. Derivatives of the loss may be computed via JAX automatic differentiation. On quantum hardware, exact differentiation schemes such as parameter-shift rule or its generalized version (as described with reference to **Fig. 18**) can be used.

Gradient evaluation is needed for training NNs in general. Since QNNs can be thought of as a function $f_\theta$, approaches for differentiation may be used. In classical ML, automatic differentiation (AD) is used, which that exploits the chain rule and stores the intermediate values of sub functions (the layers) of the ML model to evaluate the gradients of $f_\theta$. This approach has an advantageous scaling that enables training very large classical models and, hence, is always used for the differentiation of classical layers in this work. In case of QNNs, an efficient approach has been developed, beyond finite differences, for gradient evaluation. Due to the trigonometric relations within the unitaries associated with single-qubit rotations, $U_{sqr} = e^{-\frac{i}{2}\theta\hat{O}}$ (that include single-qubit Pauli generators $\hat{O}$), it is possible to derive a simple and exact derivative formula which is similar to finite differences, referred to as the Parameter-Shift Rule (PSR):

$$\nabla_\theta f_\theta(n) = \frac{f_{\theta+\pi/2}(n) - f_{\theta-\pi/2}(n)}{2}. \qquad (D1)$$

The Generalized PSR (GPSR) rule, which is needed for general multi-qubit generators, is described hereunder with reference to **Fig. 18.**

The loss function may be minimized to obtain the optimal model parameters $\theta_{opt} = argmin_\theta \mathcal{L}(\theta)$ that define the optimal XC correlation functional. A classical optimizer such as Adam of L-BFGS-B may be used to find the optimzal parameters. It is noted however that the embodiments in this application may also be performed based on gradient free optimization methods.

At each converged SCF calculation, the gradient of the loss $L$ may be computed based on automatic differentiation or the parameter-shift rule when using a quantum computer. The gradient is then provided to the classical optimizer (Adam or L-BFGS-B). The learning rate for the Adam optimizer can be decreased from $10^{-2}$ to $10^{-4}$ as the training progresses to fine tune the results. Otherwise, L-BFGS-B and Adam are used with the standard hyperparameters. To converge the training, it is possible to use a combination of Adam at first to ensure the escape of local minima due to the stochastic direction of the gradient in Adam and then switch to L-BFGS-B to follow the actual gradient, in order to achieve accurate results.

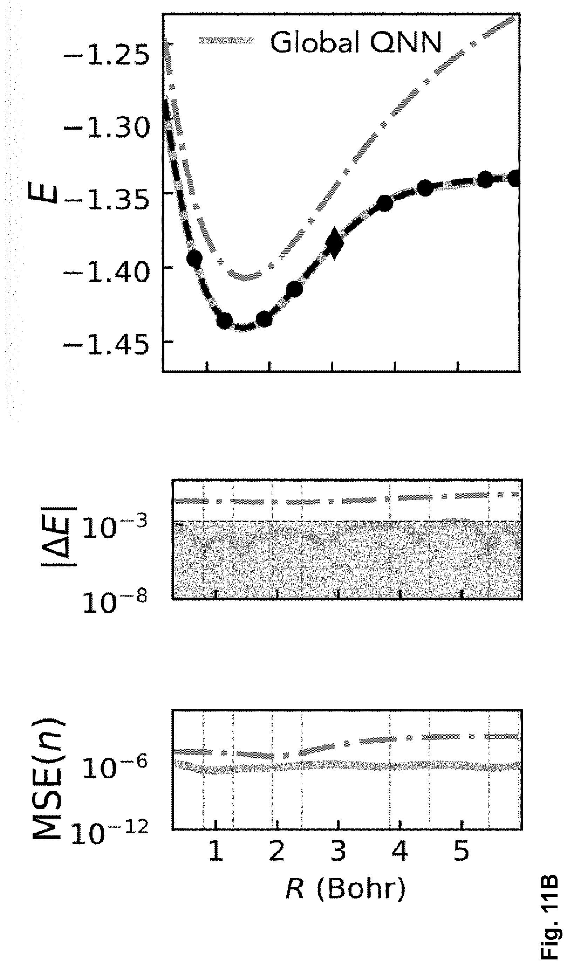

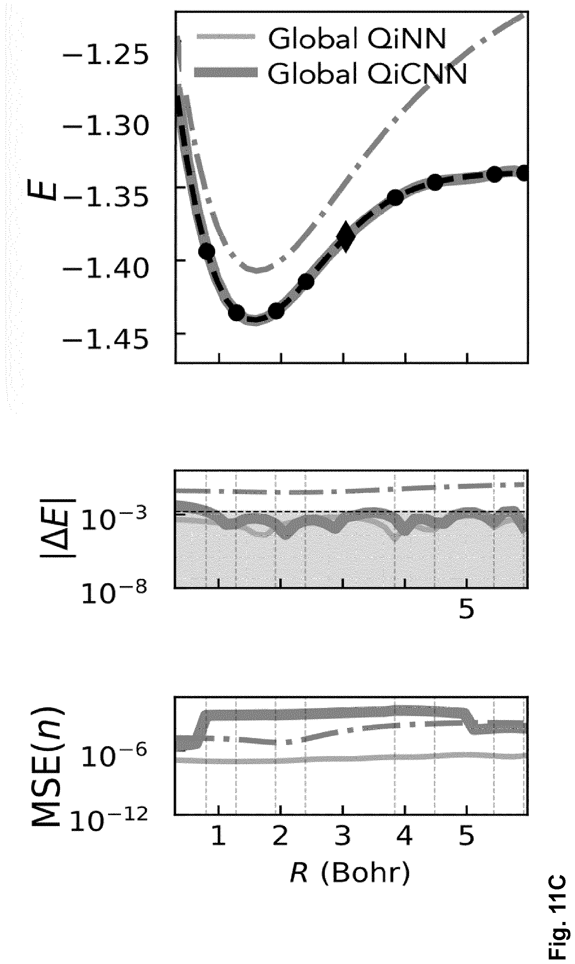

The experimental data illustrating the performance of the different models are illustrated in **Fig. 10** and **11.** All models have been evaluated using a 1D framework. The training and test dataset from DMRG simulations of Li et al. have been used, where $N_{grid} = 513$ fixed for all the systems. For all the results, a density error in terms of the mean squared error (MSE) has been defined.

For the MLP neural networks that were used for the experimental data shown in **Fig. 10,** an expressive model that has been used to evaluate the approaches of local and global embeddings. To that end, in all MLP models, 2 hidden layers with 513 neurons have been used due to the number of grid points to achieve enough expressivity. The Local MLP model has a single input and output neuron to infer the local XC energy based on corresponding density. In **Fig. 10,** the local MLP improves on the LDA DFT results in terms of overall energy error, however is not sufficient to improve on the density. The restriction local information is limiting the capacity of the model to improve. Hence, the best trade-off of energy-density error that this model is capable of is observed.

The experimental results of the quantum models are shown in **Fig. 11A-11C.** In order to evaluate if local quantum models can improve on the local MLP of Fig. **10,** the quantum models (local QNNS) employ one or more Chebyshev feature maps (ChQNN) or one or mor product feature maps (PrQNN), respectively. Here $N_q = 4$ qubits, $N_l = 1$ (no data reuploading is used) and $N_d = 8$ layers for the variational ansatz. As shown in **Fig. 11A,** the results in terms of non-parallelity error (NPE) of the energy is similar to the local MLP model. The energy and density profile of local quantum and classical models are qualitatively similar, as expected due to locality of both models. It is therefore necessary to verify if including more information in the quantum models brings benefits in terms of lower energy error $|\Delta E|$ and MSE of the density profile compared to XC functionals that are determined based on classical MLP type neural networks.

As shown in **Fig.** 10 to that end, a global version of the MLP neural network with $N_{grid} = 513$ input neurons has been evaluated. This allows the model to consider the whole density to infer the XC energy via the global embedding. Hence, the globa MPL neural network has one output neuron. This results in $\sim 25 \cdot 10^4$ parameters. As shown in **Fig. 10,** the energy profile of the global MPL neural network is significantly improved in that it closely follows the reference (lower error with respect to the reference) in comparison to all other models. However, the KSR model may be tailored to any locality. Similarly to MLP, the global version of KSR model (see equation (12) for global KSR model) performs best and generalizes in a single molecule even based pm only 2 data points (in this example 8 samples were used). Using the same density initialization as for global MLP, global KSR model struggled to generalize correctly, leading to MSE on the electron density comparable to the local density approximation (LDA) models, despite rendering chemically accurate energies. The global KSR model is however significantly more parameter-efficient, having only $\sim 10^3$ parameters versus $\sim 25 \cdot 10^4$ compared to the global MLP models, which are generally known to overfit the training data. In addition, given a good initialization, the global KSR model is capable to learn the dissociation based on only 2 data points. Generally, one may conluded based on the experimental data that models with global embedding, the results are similar or better compared to local LDA-like functionals, at least for the total energies.

The models provide smooth energy profiles due to regularization capabilities of the used differentiable density functional algorithm. It is clear that 8 data points, sampling low to strongly correlated regimes of the hydrogen molecule, are sufficient to generalize correctly within a single molecule provided the model has enough expressivity. In general, all the models show characteristic dips in energy errors at the training data.

Since local QNNs are not capable of correctly fitting the training data, architectures may be used that allow to embed $L$ data points and hence approach non-local functionals. To that end, a global QNN model is used, which is adapted to embed $L$ data points in a single QNN with $N_q = 6$ qubits, $N_l = 1$ (no data reuploading) and $N_d = 8$ variational layers. This model is then applied onto $N_{grid}/L$ different batches. The results are combined via a single-layer MLP with 171 input neurons and 1 output for the global embedding. The experimental data are shown in **Fig. 11B.** This architecture allows to embed chunks of data into a quantum model with limited number of qubits. By combining such outputs, a fully quantum-enhaned global functional may be obtained. The model has only $\sim 1000$ trainable parameters, which is orders of magnitude smaller than the global MLP for comparison since both are composed from most general quantum/classical layers. Note that an accurate objective comparision of the hybrid quantum model with the KSR model is not possible as the latter possesses numerous inductive biases such as the self-interaction gate and the softly imposed negativity of XC energy. Nevertheless, the global QNN of **Fig. 11B** show that good results are obtained compared to the other models..

**Fig. 11C** depicts a quantum model using amplitude embedding as a feature map. Here a DQC is used with $N_q = 9$ qubits, $N_l = 1$ and $N_d = 10$. This model may be referred to as the global QiNN model since differentiating amplitude embedding exactly is not feasible and in general hard to embed the classical data (density) exactly. The number of qubits was chosen to fit all the elements of the electron density **n** exactly as a state-vector. The data in **Fig. 11C** show that the results for the global QiNN model are in good agreements with the reference both in chemical accuracy and overall density error which shows that this model provides a significant improvement over the LDA models. Notably, the outcome is very similar to Global QNN, which is simple to implement on near-term devices. This model architecture, despite producing satisfactory results, does not produce a significant advantage over the hybrid QNN of **Fig. 11B.** It can however serve for theoretical studies since only quantum layers are employed, dissociating it completely from classical models.

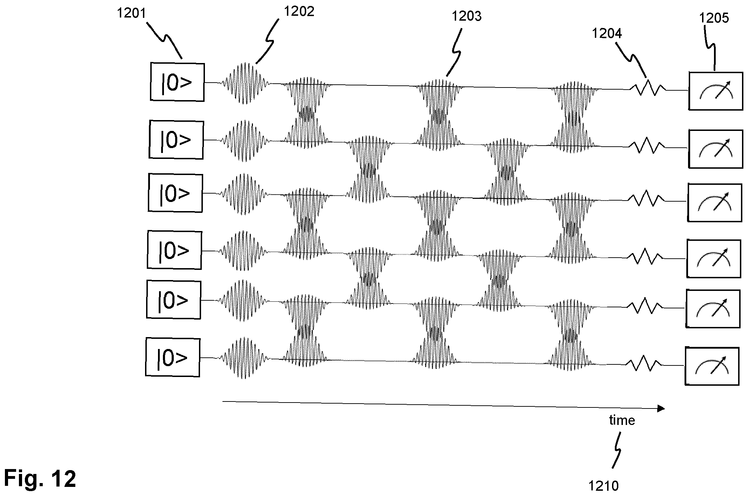

**Fig. 12** is a hardware-level schematic of the actions effectuating the logical operations as defined in a quantum circuit, e.g. DQC quantum circuit, as described with reference to the embodiments of this applications. Unitary operators, e.g. those to encode the feature map and derivatives thereof, can be decomposed into a sequence of logical gate operations. These logical gate operations are transformations in a quantum Hilbert space over the qubits that encode an input parameter. In order to transform the internal states of these qubits, a classical control stack may be used to send pulse information to a pulse controller that affects one or more qubits. The controller may send a sequence of such pulses in time and for each qubit independently. An initialization pulse is used to initialize the qubits into the $|0\rangle$ state **1202.** Then, for example a series of single-qubit pulses may be sent to the qubit array in **1204,** which may represent a single-layer feature map. Additionally, two-qubit or multi-qubit pulse sequences can be used to effectively entangle multiple qubits of the feature map. The duration, type, strength and shape of these pulses determine the effectuated quantum logical operations. **1208** indicates a 'break' in the depicted timeline, which means the sequence of gates may be repeated in a similar fashion in the direction of the time axis **1212.** At the end of the pulse sequences, one or more of the qubits are measured **1210.**

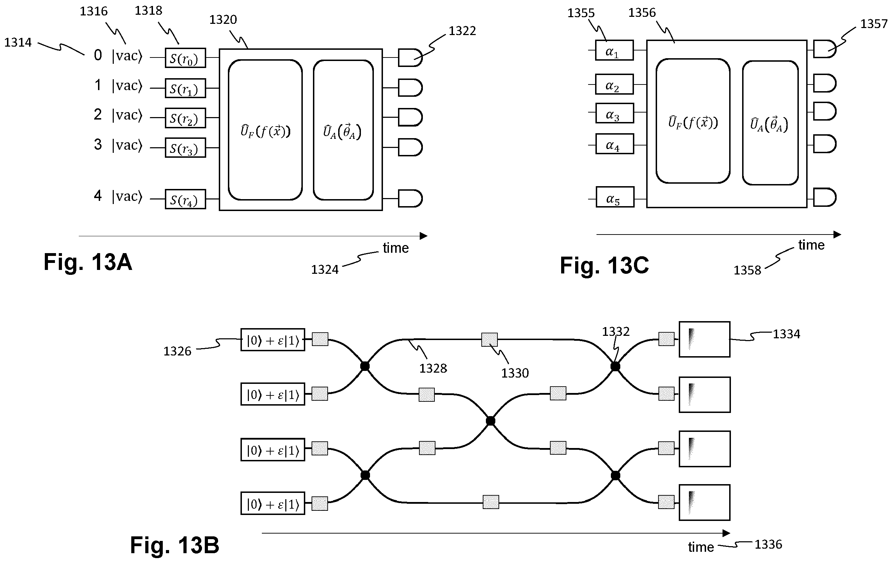

**Fig. 13A** is a hardware-level schematic of a photonic/optical quantum processor which can be used for implementing quantum models comprising trainable feature maps as described with reference to the embodiments in this application. As known from Gan et al, Fock state-enhanced expressivity of quantum machine learning models, EPJ Quantum Technology 2022, https://doi.org/10.1140/epjqt/s40507-022-00135-0 feature maps for encoding classical features or data may also be implemented based on photonic circuitry. Unitary operators, e.g. those used to encode the quantum feature map and derivatives thereof, can be decomposed into a sequence of optical gate operations. These optical gate operations are transformations in the quantum Hilbert space over the optical modes. In order to transform the internal states of these modes, a classical control stack is used to send pulse information to a pulse controller that affects one or more modes. The controller may formulate the programmable unitary transformations in a parametrised way.

Initially the modes **1314** may all be in the vacuum state **1316,** which are then squeezed to produce single-mode squeezed vacuum states **1318.** The duration, type, strength and shape of controlled-optical gate transformations determine the effectuated quantum logical operations **1320** which may define one or more feature maps associated with a first generator Hamiltonian $\vec{G}_F(f(\vec{x}))$ and, optionally, one or more variational quantum circuits (parameterized ansatze) associated with a parameterized Hamiltonian $\hat{G}_A(\vec{\theta}_A)$. At the end of the optical paths, one or more modes may be measured using a known optical measurement technique **1322,** including but not limited to photon-number resolving, a Fock basis measurement, tomography or threshold detectors.

**Fig. 13B** is a hardware-level schematic of the actions effectuating the logical operations shown in circuit diagrams specified for a Gaussian boson sampling device. Unitary operators, e.g. those used to encode the quantum feature map and derivatives thereof, can be decomposed into a sequence of optical gate operations. These optical gate operations are transformations in the quantum Hilbert space over the optical modes. In order to transform the internal states of these modes, a classical control stack may be used to send information to optical switches and delay lines. The controller may formulate the programmable unitary transformations in a parametrised way.

Initially the modes **1326** may all be in a weak coherent state, which is mostly a vacuum state with a chance of one or two photons and negligibly so for higher counts. Subsequently, the photons travel through optical waveguides **1328** through delay lines **1330** and two-mode couplers **1332** which can be tuned with a classical control stack, and which determines the effectuated quantum logical operations, which may which may define one or more feature maps associated with a first parameterized generator Hamiltonian and, optionally, one or more variational quantum circuits (parameterized ansatze) associated with a second parameterized Hamiltoning. At the end of the optical paths, one or more modes are measured with photon-number resolving **1334,** or threshold detectors.

**Fig. 13C** is a hardware-level schematic of the actions effectuating the logical operations specified for a photonic/optical quantum processor. The quantum model can be decomposed into a sequence of optical gate operations. These optical gate operations are transformations in the quantum Hilbert space of the photons. In order to transform the internal states of these photons, a classical control stack is used to send information to a universal multiport interferometer. The controller may formulate the programmable unitary transformations in a parameterized way. Initially the photons **1355** are in Fock states, weak coherent states or coherent states. The duration, type, strength and shape of controlled-optical gate transformations determine the effectuated quantum logical operations **1356** , which may which may define one or more feature maps associated with a first parameterized generator Hamiltonian and, optionally, one or more variational quantum circuits (parameterized ansatze) associated with a second parameterized Hamiltoning. At the end of the optical paths, the modes are measured with photon-number resolving, Fock basis measurement **1357,** tomography or threshold detectors.

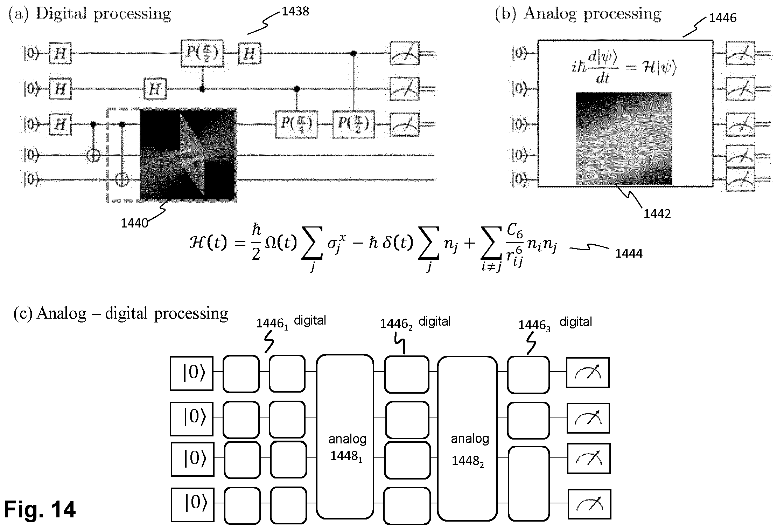

**Fig. 14** is a hardware-level schematic of actions effectuating the logical operations shown in a quantum circuit which may be executed on a so-called neutral-atom-based quantum computer. On this type of hardware, unitary operators, e.g. those used to encode the quantum feature map and derivatives thereof, can be decomposed in two different kinds of operations: digital or analog. Both of these operations are transformations in the quantum Hilbert space over atomic states.

Schematic (a) of **Fig. 14** depicts a digital quantum circuit **1438,** wherein local laser pulses may be used to individually address neutral atoms to effectuate transitions between atomic states which effectively implement sets of standardized or 'digital' rotations on computational states in accordance with the quantum circuit. Digital gates may include any single-qubit rotations, and a controlled-pauli-Z operation with arbitrary number of control qubits. Additionally, such digital gate operations may also include 2-qubit or even multi-qubit operations.

Schematic (b) of **Fig. 14** depicts an analog mode **1446** of operation of a neutral-atom-based quantum computer, wherein a global laser light pulse may be applied to groups of, or all, atoms at the same time, with certain properties like detuning, Rabi frequencies and Rydberg interactions to cause multi-qubit entanglement thereby effectively driving the evolution of a Hamiltonian **1444** of the atomic array in an analog way. The combined quantum wavefunction evolves according to Schrödinger's equation, and particular, unitary operators $\hat{\mathcal{U}} = e^{-i \hat{\mathcal{H}} t}$, where $\hat{\mathcal{H}}$ denotes the Hamiltonian and $t$ the time, can be designed by pulse-shaping the parametrised coefficients of the Hamiltonian in time. This way, a parametric analog unitary block can be applied, which entangles the atoms and can act as a variational ansatz, or a feature map, or other entanglement operation.

The digital and analog modes shown in this figure may be combined or alternated, to yield a combination of the effects of each as shown in schematic (c) of Fig. **14.** This figure depicts an example of such digital-analog quantum circuit, including digital blocks **1246**$_{1-3}$ of digital qubit operations (single or multi-qubit) and analog blocks **1448**$_{1-3}$, wherein the operations of the digital and/or analog blocks may be used to implement one or more trainable feature maps as described with reference to the embodimebts in this application.

It can been proven that any computation can be decomposed into a finite set of digital gates, including always at least one multi-qubit digital gate (universality of digital gate sets). This includes being able to simulate general analog Hamiltonian evolutions, by using Trotterization or other simulation methods. However, the cost of Trotterization is expensive, and decomposing multi-qubit Hamiltonian evolution into digital gates is costly in terms of number of operations needed.

Digital-analog circuits define circuits which are decomposed into both explicitly-digital and explicitly-analog operations. While under the hood, both are implemented as evolutions over controlled system Hamiltonians, the digital ones form a small set of pre-compiled operations, typically but not exclusively on single-qubits, while analog ones are used to evolve the system over its natural Hamiltonian, for example in order to achieve complex entangling dynamics.

It can be shown that complex multi-qubit analog operations can be reproduced/simulated only with a relatively large number of digital gates, thus posing an advantage for devices that achieve good control of both digital and analog operations, such as neutral atom quantum computer. Entanglement can spread more quickly in terms of wall-clock runtime of a single analog block compared to a sequence of digital gates, especially when considering also the finite connectivity of purely digital devices. Hence, in an embodiment, the quantum models described in this disclosure for representing XC functionals may include digital-analog quantum circuits.

Further, digital-analog quantum circuits for a neutral quantum processor that are based on Rydberg type of Hamiltonians can be differentiated analytically so that they can be used in variational and/or quantum machine learning schemes, including the differential quantum circuit (DQC) schemes and the kernel methods as described in this application. In order to transform the internal states of these modes, a classical control stack is used to send information to optical components and lasers. The controller may formulate the programmable unitary transformations in a parametrised way.

At the end of the unitary transformations, the states of one or more atoms may be read out by applying measurement laser pulses, and then observing the brightness using a camera to spot which atomic qubit is turned 'on' or 'off', 1 or 0. This bit information across the array is then processed further according to the embodiments.

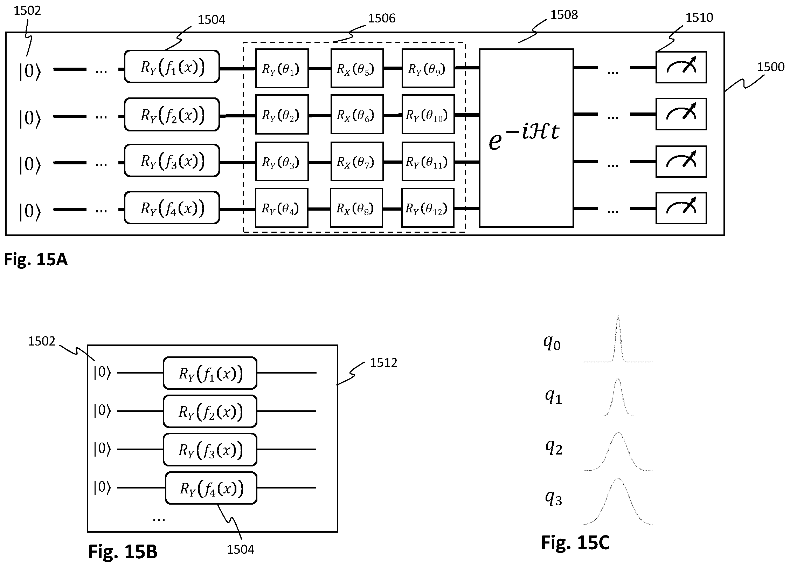

**Fig. 15A** depicts a system describing a digital-analog implementation of a quantum circuit as described with reference to the embodiments in this application. In particular, **Fig. 15A** represents a digital-analog implementation where the qubit interactions can be switched on and off and there exists single-qubit addressability for the application of single-qubit gates. In order to transform the internal states of these qubits, a classical control stack may be used to send information to a controller that affects one or more qubits. The controller may send multiple information carriers (e.g., pulses) in time and for each qubit independently. An initialization protocol may be used to initialize the qubits, in this example into the $|0\rangle$ state **1502**. The current example shows only four qubits, but the same principles may be extended in a straightforward manner to systems with more (or less) than four qubits.

Then, a quantum feature map may be applied **1504** to encode quantum information, e.g., an input variable, into a Hilbert space associated with the quantum processor. Following application of the feature map, a variational Ansatz may be applied, implemented as a variational quantum circuit **1506**. In the current example, the variational Ansatz comprises three single-qubit gates $R_Y$ - $R_X$ - $R_Y$ with different rotational angles $\theta_i$. These single-qubit gates are typically sets of standardized or 'digital' rotations on computational states applied to different qubits with different rotation angles/parameters. These digital gates include any single-qubit rotations according to the $\theta_i$ argument of the rotation. A single rotation/single-qubit gate is not sufficient to perform arbitrary rotation regardless of the parameter/angle since it can only rotate over a single axis. In order to accomplish a general rotation block, three gates in series with different rotation angles/parameters can be used, in this example represented by a block of single-qubit gates **1506**.

Then, entanglement in this digital-analog approach may be generated by a wavefunction evolution **1508,** described by the block $e^{-i \mathcal{H} t}$. During this evolution, the qubits are interacting amongst themselves, letting the system evolve for a specified amount of time. This process produces the necessary entanglement in the system. The combined quantum wavefunction evolves according to Schrödinger's equation, and particular unitary operators $\hat{\mathcal{U}} = e^{-i \mathcal{H} t}$ in which $\mathcal{H}$ is the Hamiltonian of the system (for example, for neutral atoms the Hamiltonian that governs the system is $\mathcal{H} =$

$$\sum_{j \neq k} \left( \frac{c_6}{r_{j k^6}} \right) * \hat{n}_j * \hat{n}_k$$

with $\hat{n}$ being the state occupancy of the Rydberg atoms), and $t$ is the evolution time. In this way, a parametric analog unitary block can be applied, which entangles the atoms and can act as a variational Ansatz.

After the evolution of the wavefunction **1508,** another set of single-qubit gates may be applied, similar to the process described in the block of gates $R_Y$ - $R_X$ - $R_Y$ **1506**. Then, the wavefunction may be evolved once more, and finally a measurement in the computational basis occurs as described in **1510**. Optionally, additional steps may be included, represented by the ellipses, e.g., before, after, or in between the blocks shown. For example, a different initial state than 10) may be prepared prior to application of the feature map. Furthermore, the blocks can be executed in a different order, for instance, in some embodiments, block **1508** might precede block **1506**. One or more of the blocks **1504-1508,** or variations thereof, may be repeated one or more times prior to the measurement **1510**.

**Fig. 15B** represents a trainable quantum feature map **1512** which applies single-qubit rotations to qubits **1502** with (typically) different rotation angles **1504**. In this example, single qubit rotations **1504** (here chosen as $R_Y(f_i(x))$) act on each qubit individually and are parametrized by a variable $x$ which may be encoded according to one or more encoding functions $f_i(x)$. For a non-linear feature map encoding, the variable $x$ may be used as an angle of rotation. The application of one rotation operation to each qubit in a register may be referred to as a single layer of rotation operations. This type of encoding of classical information into quantum states wherein a data vector is represented by the angles of a quantum state. This type of encoding can be found in many quantum machine learning (QML) algorithms, with the main advantage being that it only requires $n = \log NM$ qubits to encode a dataset of $M$ inputs with $N$ features each. Thus, considering an algorithm that is polynomial in $n$, it has a logarithmic runtime dependency on the data size.

Fig. 15C illustrates different pulses (potentially differing in pulse duration and/or amplitude/height) that can be sent to the quantum circuit (different qubits) to encode the classical information. The angle of the rotation that is described by the $f_i(x)$ dictates the duration and geometry of the pulse.

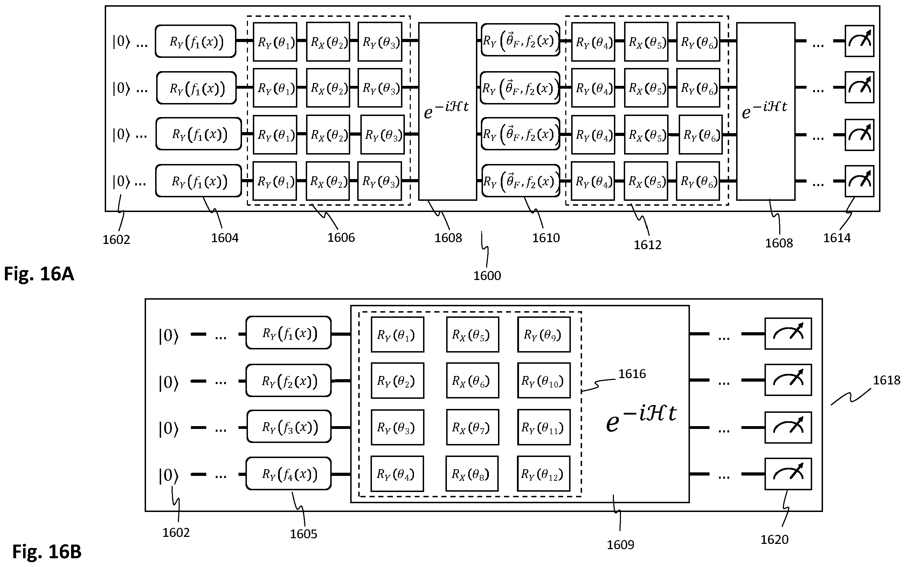

**Fig. 16A** presents a quantum circuit based on a digital-analog implementation **1600** that has no single-qubit addressability and uses a wavefunction evolution that can be turned on and off for the entanglement generation. **Fig. 16A** differs from **Fig. 15A** in that it shows a technique known as data re-uploading that alters the trainable quantum feature maps **1604, 1610.** Loading classical data into a quantum system is a non-trivial task, and uploading large amounts of data makes it an even more critical issue. However, when the case of big-data is not considered, techniques like data re-uploading can be utilized. As the name suggests, this entails uploading the classical data multiple times, and encode them via rotational angle/parameter encoding into single-qubit rotations throughout the quantum circuit. Such a data re-uploading technique may start by setting the quantum register to an initial state, e.g., the all-zero state **1602,** followed by angle encoding that is based on a feature map **1604,** in this case a tower feature map.

Following that, as already explained above with reference to **1606,** three single-qubit gates in series are used to perform a general rotation block that acts as the Ansatz **1606**. In this example the encoding is done through the $\theta_i$ variable of the $R_Y$ - $R_X$ - $R_Y$ rotations; however, any type of combined rotations of the type $(R_x, R_y, R_z)$ could be applied here. Since there is no single-qubit addressability in this example, each $R_Y$ and $R_X$ gate is applied at all qubits simultaneously with the same rotation angle $\theta_i$. Subsequently, the wavefunction is allowed to evolve for a specified amount of time **1608**.

Then the classical data are re-uploaded through angle encoding into single-qubit rotations **1610**. However, each time the (same) data are uploaded, the information is encoded using different angles than in the previous data uploading steps. For example, the amount of rotation may be doubled in each data (re-)uploading step **1604, 1610** (i.e., the rotational angle of the might be increasing as 1-2-4, etc.).

Once again, the rotations $R_Y$ - $R_X$ - $R_Y$ are applied to all qubits simultaneously with the same rotation angle for each single qubit gate, but different than **1606.** The classical information can be repeatedly encoded into the quantum feature map through the data re-uploading technique, while changing the angle of the single qubit rotational angle (**1604, 1610,** etc.) every time after the wavefunction evolution. The resulting quantum feature map can become more expressive as a tower feature map by doing this process of serial data re-uploading with different levels of rotational angles every time the re-uploading is done.

**Fig. 16B** illustrates a different (and at current technology levels more realistic) form of a digital-analog implementation which may be used in the embodiments described in this application. In this implementation, the system has single-qubit addressability, but the wavefunction evolution cannot be turned off, thus staying always on. This system requires less control over the qubit interactions (since they are always on) and only requires single-qubit addressability. The single-qubit gates are applied while the wavefunction evolution (qubit interaction) is also happening. In the shown example, the quantum circuit **1618** starts by encoding information in the 10) quantum state **1602,** followed by a trainable quantum feature map **1605.**

Then, a variational Ansatz is applied, comprising both wavefunction evolution $e^{-i\mathcal{H}t}$ **1609** and simultaneous application of a block of single-qubit gates **1616.** After multiple repetitions of block **1616,** each time with different rotational angles at the single-qubit gates, the circuit ends with a measurement in the computational basis **1620.**

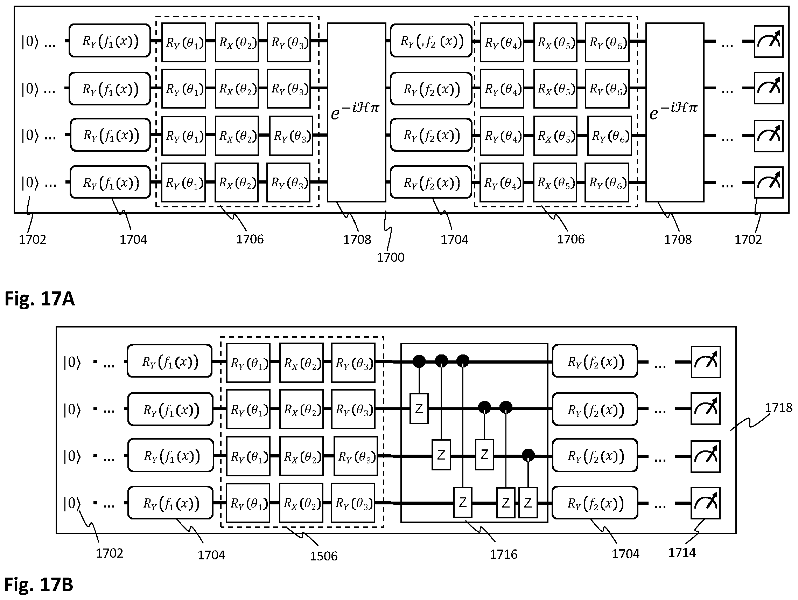

**Fig. 17A** illustrates a quantum circuit **1700** that implements a digital-analog implementation with single-qubit addressability, wherein the wavefunction evolution (e.g., atom interactions) can be turned on and off. This quantum circuit is equivalent to the quantum circuit in **Fig. 16A,** with the time of the wavefunction evolution **1608** being $t = \pi$ (in dimensionless coordinates), while all other operations are the same as the ones described in **1500.** The same technique of data re-uploading with a tower feature map **1704, 1510** and the same Ansatz comprising single-qubit gates **1706,1712** followed by wavefunction evolution **1708** are used.

The reason that such a specific time period for the wavefunction evolution **1708** is selected, is due to the fact that such evolution results in the application of Controlled-Z (CZ) gates between pairs of qubits. The benefit of describing the wavefunction evolution as a set of CZ gates lies in the fact that this Digital-Analog implementation of a quantum circuit resembles the structure of the equivalent Digital quantum circuit, which instead of the operation **1708** performs a set of CNOT gates between pairs of qubits included in the quantum circuit. This allows more straightforward comparisons to be drawn between the Digital and Analog implementation of such feature maps, and to evaluate their relative performance.

**Fig. 17B** illustrates a quantum circuit that implements a digital-analog implementation with single-qubit addressability **1718,** similar to **1700,** but with the entanglement generated via CZ gates between the qubits, resembling the atom interaction / entanglement generation.

**Fig. 18** presents a workflow for generalized circuit differentiation. In Quantum Machine Learning (QML) algorithms, various quantum feature maps and Parametric Quantum Circuits (PQCs) are typically utilised to express the circuit required to solve a particular problem. These quantum circuits contain tuneable parameters that use a classical optimizer to find the optimal values of the parameters that exist for that formulation. The differentiation required for most parameter optimization methods can be achieved in various ways, with the most common one being the Parameter Shift Rule (PSR). An example of such kind of differentiation for quantum feature maps is presented in **Fig. 18.**

One way to calculate analytic derivatives of quantum circuits is by measuring overlaps between quantum states.

Analytic derivatives can be used for differentiating unitaries like the one presented in **1804,** e.g., $\widehat{\mathcal{U}} = e^{-ix\hat{G}(\theta,x)/2}$ generated by arbitrary Hermitian generator $\hat{G}$. However, in order to have a less resource-intensive method for differentiation, the parameter shift rule (PSR) was proposed. The PSR algorithm can provide analytic gradient estimations through measurement of the expectation values, with gate parameters being shifted to different values. The PSR algorithm is much used to perform differentiation for QML algorithms; however, it is only valid for a specific type of generators, viz., generators that are involutory or idempotent. This is because the full analytic derivative only requires 2 measurements of expectation values (2 unique eigenvalues). Although this simplifies the differentiation protocol, it also restricts it to only work for a certain type of generators.

Therefore, as an improvements and generalizations of the PSR algorithm, in **1800** a scheme for a Generalized Parametric Shift Rule (GPSR) algorithm is shown. Such approach allows for differentiating generic quantum circuits with unitaries generated by operators with a rich spectrum (i.e., not limited to idempotent generators). The GPSR algorithm is based on spectral decomposition, and showcases the role of the eigenvalue differences (spectral gaps) during differentiation for the generator spectrum. Such approach works for multiple non-degenerate gaps, in contrast to the PSR that is only valid for involutory and idempotent operators with a single unique spectral gap.

A workflow for generalized circuit differentiation can be described as follows: create a quantum circuit that encodes the function $f$ similar to **1800** including single- and multi-qubit gates **1802.** Pick a unitary operator such as $\widehat{\mathcal{U}}(x) =$ exp(-i $x$ $\hat{G}$/2) **1804** that is parametrized by some tuneable parameter $x$, and study the spectrum of its generator $G$. Then, using unique and positive spectral gaps, a system of equations that includes the spectral information can be created, alongside the calculated parameter shifts, and the measured function expectation values as shifted parameters. The solution of such system can provide the analytical derivative for any general generator G and thus perform GPSR. GPSR is described in more detail in Kyriienko et al., 'Generalized quantum circuit differentiation rules', Phys. Rev. A 104 (052417), which is hereby incorporated by reference.

The techniques of this disclosure may be implemented in a wide variety of devices or apparatuses, including a wireless handset, an integrated circuit (IC) or a set of ICs (e.g., a chip set). Various components, modules, or units are described in this disclosure to emphasize functional aspects of devices configured to perform the disclosed techniques, but do not necessarily require realization by different hardware units. Rather, as described above, various units may be combined in a hardware unit or provided by a collection of interoperative hardware units, including one or more processors as described above, in conjunction with suitable software and/or firmware.

The terminology used herein is for the purpose of describing particular embodiments only and is not intended to be limiting of the invention. As used herein, the singular forms "a," "an," and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

The corresponding structures, materials, acts, and equivalents of all means or step plus function elements in the claims below are intended to include any structure, material, or act for performing the function in combination with other claimed elements as specifically claimed. The description of the present invention has been presented for purposes of illustration and description, but is not intended to be exhaustive or limited to the invention in the form disclosed. Many modifications and variations will be apparent to those of ordinary skill in the art without departing from the scope and spirit of the invention. The embodiment was chosen and described in order to best explain the principles of the invention and the practical application, and to enable others of ordinary skill in the art to understand the invention for various embodiments with various modifications as are suited to the particular use contemplated.

**Claims**

1. Method of determining an exchange-correlation functional for computing electronic properties of a molecular system using a hybrid data processor comprising a classical computer and a quantum computer, the method comprising:

   receiving or determining reference data comprising synthetically computed and/or real-world measured reference electron-orbital features, preferably reference electron densities and associated reference ground state energies, for a plurality of grid points, the reference electron-orbital features being associated with different molecular reference systems;
   determining a first electron density of a molecular system for which reference electron-orbital features are available;
   computing a second electron density for each of the grid points based on: the first electron density, an exchange correlction energy computed by a variational quantum model using the first electron density as input; and, a density functional algorithm, preferably a self-consistent density functional algorithm; and,
   if the difference between the second electron density and the first electron density is smaller than a predetermined delta value, then updating variational parameters of the variational quantum model based on a training loss, wherein a loss function is used to compute the training loss based on the second electron density and a reference electron density respectively.

2. Method according to claim 1 wherein the variational quantum model is represented by a quantum circuit defining gate operations, the quantum circuit defining a quantum neural network comprising one or more feature maps and one or more parameterized variational circuits.

3. Method according to claim 2 wherein the variational quantum model further comprises one or more classical neural networks configured, wherein the updating of the variational parameters includes updating the weights of the classical neural network.

4. Method according to claims 2 or 3 wherein computing the exchange correlation energy includes:

   using the one or more feature maps to encode the first electron density at at least part of the grid points in the Hilbert space; and,
   executing the quantum circuit on quantum elements of the quantum computer;
   measuring the state of the quantum elements to obtain an exchange correlation energy.

5. Method according to claim 4 wherein executing the quantum circuit includes:

   translating quantum operations of the quantum circuit into control signals;
   controlling the quantum elements of the quantum computer based on the control signals.

6. Method according to claims 4 or 5 wherein executing the quantum circuit includes:

   determining classical measurement data by measuring the state of the quantum elements of the quantum computer;
   determining the exchange-correlation energy based on the classical measurement data.

7. Method according to any of claims 1-6 wherein computing predicted electron-orbital features includes:

   determining an exchange-correlation potential based on the derivative of the predicted exchange correlation energy with respect to the electron density; and,
   determining Coulomb and Hartee potentials using the first electron density;
   computing the second electron-orbital features based on the self-consistent density functional algorithm using the Coulomb and Hartee potentials and the exchange-correlation potential as input.

8. Method according to any of claims 1-7 wherein the one or more feature maps are trainable feature maps, the trainable feature maps including one or more unitary operators defining a time evolution over a Hamiltonian, preferably a generator Hamiltonian, applied to the quantum elements of the quantum register, the Hamiltonian evolution being parameterized by variational parameters.

9. Method according to any of claims 1-8 wherein quantum circuit comprises digital quantum gate operations, preferably digital single quantum gate operations, and one or more analog quantum gate operations configured to entangle different quantum elements of the quantum computer and to evolve a Hamiltonian associated with quantum elements in time;

10. Method according to any of claims 1-9 further comprising:
    if the difference between the second electron-orbital features and the first electron-orbital features is larger than the predetermined delta, computing third electron-orbital features for each of the grid points based on: the second electron-orbital features, a predicted exchange correlction energy computed by the quantum model using the second electron-orbital features as input; and, the density functional algorithm.

11. Method according to any of claims 1-10 wherein further comprises:

    computing a ground state energy associated with the second electron density; and,
    further computing the loss value based on the ground state energy and a reference ground state energy associated with the reference electron density.

12. Method according to any of claims 1-11 wherein at least part of the variational parameters are adjusted using a classical optimizer, preferably a gradient-based optimizer.

13. Method according to any fo claims 1-12 wherein the one or more electron densities are encoded based rotation operations and a non-linear encoding function, preferably the non-linear encoding function including a Chebyshev polynomial.

14. Method according to any of claims 1-13 wherein the variational quantum model comprises one or more differentiable quantum circuits (DQCs), the one or more differentiable quantum circuits including one or more derivative features maps for encoding the one or more derivatives of the exchange-correlation functional.

15. Method according to any of claims 1-14 wherein the quantum computer is implemented based on at least one of: a neutral atom quantum device, an ion-based quantum device, an optical and photonic quantum device, a superconducting quantum device, a silicon based quantum device, a diamond N-V centre quantum device and/or a gaussian boson sampling device.

16. A hybrid data processor for determining an exchange-correlation functional for computing electronic properties of a molecular system, the hybrid data processing comprising a classical computer and a quantum computer, the hybrid data processing being configured to:

receiving or determining reference data comprising synthetically computed and/or real-world measured reference electron-orbital features, preferably reference electron densities and associated reference ground state energies, for a plurality of grid points, the reference electron-orbital features being associated with different molecular reference systems;

determining a first electron density of a molecular system for which reference electron-orbital features are available;

computing a second electron density for each of the grid points based on: the first electron density, an exchange correlction energy computed by a variational quantum model using the first electron density as input; and, a density functional algorithm, preferably a self-consistent density functional algorithm; and,

if the difference between the second electron density and the first electron density is smaller than a predetermined delta value, then updating variational parameters of the variational quantum model based on a training loss, wherein a loss function is used to compute the training loss based on the second electron density and a reference electron density respectively.

17. A computer program or suite of computer programs comprising at least one software code portion or a computer program product storing at least one software code portion, the software code portion, when run on a hybrid data processing system comprising a classical computer and a quantum computer comprising a hardware quantum register, being configured for executing the method steps according any of claims 1-15.

**Fig. 1**

**Fig. 2A**

Fig. 2B

Fig. 3

**Fig. 4A**

Fig. 4B

Obtain (quantum-enhanced) functional $f_{\theta*}$ ~ 414

Evaluate on unseen molecules $E$ Bond distance $n$ Integration grid ~ 416

receiving or determining a training dataset comprising synthetically generated and/or real-world measured reference energies and reference electron densities for a plurality of grid points, the reference energies and reference electron densities being associated with different molecular reference systems;

502

receiving or determining information regarding a quantum circuit defining gate operations of a parameterized quantum model, the quantum circuit comprising one or more feature maps for embedding one or more electron densities into the Hilbert space of the quantum computer and comprising one or more parameterized variational quantum circuits;

504

training the parameterized quantum model as an exchange-correlation functional, the training including embedding for at least part of the grid points first electron densities in the one or more feature maps, executing the gate operations of the quantum circuit using quantum elements of the quantum computer to compute an exchange-correlation energy, computing a second electron density based on the exchange correlation energy and a self-consistent density functional algorithm; and, updating the parameters of the variational quantum circuit based on a training loss, the training loss being computed based on the second electron density and a reference electron density.

506

**Fig. 5**

Fig. 6

706

$716 \quad \vec{\theta} \rightarrow \vec{\theta'}$

for $x_j$ in $X$:

(variational) feature map with generator $\hat{G}(\phi(\vec{x}), \vec{\gamma}, \vec{\theta}_F)$

605

variational ansatz $\vec{\theta}_A$

708

hardware measurements

$\langle C(x, \vec{\theta}) \rangle \quad |\langle \psi(x_j) | C(y_j) \rangle|$

$x \mapsto x_j$

710

derivative (variational) feature map

707

variational ansatz

709

hardware measurements

$\dfrac{d\langle C(x, \vec{\theta}) \rangle}{dx} \quad \dfrac{d|\langle \psi(x_j) | C(y_j) \rangle|}{dx}$

$x \mapsto x_j$

711

704

compute quantities required by optimizer e.g., $L$ and loss gradient $dL/d\theta$

$\{ \vec{\theta}_F, \vec{\theta}_A, u(x, \vec{\theta}_F, \vec{\theta}_A) \}$

712

optimization algorithm

714

706

702

**Fig. 7A**

EP 4 629 143 A1

718

$|0\rangle$
$|0\rangle$
$|0\rangle$
$|0\rangle$

721

725

722

724

720    723

**Fig. 7B**

**Fig. 7B**

**Fig. 8A**

**Fig. 8C**

**Fig. 9**

902 — Input: DFT problem for molecular system

904 — select parametric quantum model for the XC functional

906 — determine each term in latent space

920 — determine unknown function as parametrized wavefunction

922 — determine derivative(s) as modified parametrized wavefunction

924 — determine known functions of x into latent space

908 — optimize the model in the latent space by running the DFT solver (i.e., KS-DFT uses the model during KS iterations and loss gradients are computed)

916 — Variational Solver Minimizing Loss

918 — Measure overlaps on Quantum Device

914 — Evaluate XC energy with the quantum model at solution parameters in a DFT solver

Molecular data: e.g., reference electron densities $y$ exchange-correlation potential $y' = v_{xc}$

$x$ is some input

$y_\theta(x) = \langle x | y_\theta \rangle$

$|x\rangle = \frac{1}{2^{N/2}} T_0(x)|0\rangle + \frac{1}{2^{(N-1)/2}} \sum_{k=1}^{2^N-1} T_k(x)|k\rangle$

$|y_\theta\rangle = \mathcal{U}_\theta |0\rangle$

$|y_\theta\rangle = \mathcal{U}_\theta |0\rangle \longrightarrow y_\theta(x) = \langle x | y_\theta \rangle\rangle$

$|dydx\rangle\rangle = G\mathcal{U}_\theta |0\rangle \longrightarrow y'_\theta(x) = \langle x | dydx\rangle\rangle$

Optimize the model in the latent space

$\mathcal{L}_{\text{data}}(\theta) = \sum_{x_i \in \mathcal{X}} \{y_\theta(x_i) - y_i\}^2 + \mathcal{L}_{\text{data}}^{v_{xc}}(\theta) = \sum_{x_i \in \mathcal{X}} \{y'_\theta(x_i) - y'_i\}^2$

Ref. densities    Ref. XC potential

Evaluate solution at points $x_j$ using params $\tilde{\theta}$

$\epsilon_{\text{XC}}(x_j) = y_{\tilde{\theta}}(x_j) = \langle x_j | y_{\tilde{\theta}}\rangle$    1-local XC energy

Fig. 10

Fig. 11A

Fig. 11B

Fig. 11C

**Fig. 12**

Fig. 13A

Fig. 13C

Fig. 13B

(a) Digital processing

(b) Analog processing

$$\mathcal{H}(t) = \frac{\hbar}{2}\Omega(t)\sum_{j}\sigma_j^x - \hbar\,\delta(t)\sum_{j} n_j + \sum_{i\neq j}\frac{C_6}{r_{ij}^6} n_i n_j \quad 1444$$

(c) Analog – digital processing

**Fig. 14**

EP 4 629 143 A1

Fig. 15A

Fig. 15B

Fig. 15C

Fig. 16A

Fig. 16B

**Fig. 17A**

**Fig. 17B**

Fig. 18

Europäisches
Patentamt

European
Patent Office

Office européen
des brevets

# EUROPEAN SEARCH REPORT

**Application Number**

EP 24 30 5871

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CEREZO M ET AL: "Challenges and Opportunities in Quantum Machine Learning", ARXIV.ORG, CORNELL UNIVERSITY LIBRARY, 201 OLIN LIBRARY CORNELL UNIVERSITY ITHACA, NY 14853, 16 March 2023 (2023-03-16), XP091460778, DOI: 10.1038/S43588-022-00311-3 * abstract; Sections; page 1 - page 10; figures 1-5 * | 1-17 | INV. G06N10/60 G16C10/00 G16C20/30 |
| X | THOMAS E BAKER ET AL: "Density functionals and Kohn-Sham potentials with minimal wavefunction preparations on a quantum computer", ARXIV.ORG, [Online] 17 November 2020 (2020-11-17), XP081814011, DOI: 10.1103/PHYSREVRESEARCH.2.043238 [retrieved on 2024-11-15] * abstract; Sections I-V; Appendix A-D; figure 1 * | 1-17 | |
| A | WO 2022/148847 A1 (DEEPMIND TECH LTD [GB]) 14 July 2022 (2022-07-14) * paragraph [0004] - paragraph [0025]; paragraph [0032] - paragraph [0127]; figures 1-4 * | 1-17 | **TECHNICAL FIELDS SEARCHED (IPC)** G06N G16C |
| A | MARCELLO BENEDETTI ET AL: "Parameterized quantum circuits as machine learning models", ARXIV.ORG, CORNELL UNIVERSITY LIBRARY, 201 OLIN LIBRARY CORNELL UNIVERSITY ITHACA, NY 14853, 18 June 2019 (2019-06-18), XP081383181, * abstract; Sections I-IV; figures 1-6 * | 1-17 | |

-/--

The present search report has been drawn up for all claims

| Place of search | Date of completion of the search | Examiner |
|---|---|---|
| The Hague | 19 November 2024 | Klasen, TJ |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

## EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Sheridan Evan ET AL: "Enhancing density functional theory using the variational quantum eigensolver", arXiv.org, 28 February 2024 (2024-02-28), XP093180702, DOI: 10.48550/arxiv.2402.18534 Retrieved from the Internet: URL:https://arxiv.org/pdf/2402.18534 [retrieved on 2024-11-15] * abstract; Sections 1-6; Algorithms 1 and 2 * | 1-17 | |

TECHNICAL FIELDS
SEARCHED      (IPC)

The present search report has been drawn up for all claims

| Place of search | Date of completion of the search | Examiner |
|---|---|---|
| The Hague | 19 November 2024 | Klasen, TJ |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

........................................................................................

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 30 5871

This annex lists the patent family members relating to the patent documents cited in the above-mentioned European search report.
The members are as contained in the European Patent Office EDP file on
The European Patent Office is in no way liable for these particulars which are merely given for the purpose of information.

19-11-2024

| Patent document cited in search report | Publication date | Patent family member(s) | Publication date |
|---|---|---|---|
| WO 2022148847 A1 | 14-07-2022 | CN 116783656 A<br>EP 4260326 A1<br>US 2024071577 A1<br>WO 2022148847 A1 | 19-09-2023<br>18-10-2023<br>29-02-2024<br>14-07-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2022148847 A
- WO 2022101483 A
- EP 23186558
- WO 2023170003 A
- EP 23186602

**Non-patent literature cited in the description**

- **KIRKPATRICK et al.** Pushing the frontiers of density functionals by solving the fractional electron problem. *science*, 2021, vol. 374 (6573)
- **LI et al.** Kohn-Sham equations as regularizer: building prior knowledge into machine-learned physics. *Phys. Rev. Lett.*, 2021, vol. 126, 036401
- **DSGUPTA et al.** Standard grids for high-precision integration of modern density functionals: SG-2 and SG-3. *Journal of Computational Chemistry*, 2017, vol. 38 (12)
- **LI et al.** Kohn-Sham equations as regularizer: Building prior knowledge into machine-learned physics.. *Physical review letters*, 2021, vol. 126.3, 036401
- **ZHANG et al.** *J. Chem. Phys.*, 2022, vol. 157, 204801
- **CHAI**. *J. Chem. Phys.*, 2012, vol. 136, 154104
- **R. J. BARTLETT** ; **M. MUSIAL**. *Reviews of Modern Physics*, 2007, vol. 79, 291
- **GAN et al.** Fock state-enhanced expressivity of quantum machine learning models. *EPJ Quantum Technology*, 2022, https://doi.org/10.1140/epjqt/s40507-022-00135-0
- **KYRIIENKO et al.** Generalized quantum circuit differentiation rules. *Phys. Rev. A*, vol. 104, 052417